# EUROPEAN PATENT SPECIFICATION

(11) **EP 2 503 881 B1**
(45) Date of publication and mention of the grant of the patent: **13.05.2015**
(21) Application number: 10833831.0
(22) Date of filing: 23.11.2010
(51) Int. Cl.: C07D 471/04, C07D 471/14, C07D 493/04, C07D 495/04, C07D 513/04, C07D 519/00, A61K 31/4745, A61P 31/12, A61P 31/22

(54) **FUSED TRICYCLIC COMPOUNDS AND DERIVATIVES THEREOF USEFUL FOR THE TREATMENT OF VIRAL DISEASES**
KONDENSIERTE TRIZYKLISCHE VERBINDUNGEN UND DERIVATE DAVON ZUR BEHANDLUNG VON VIRENERKRANKUNGEN
COMPOSÉS TRICYCLIQUES FUSIONNÉS ET LEURS DÉRIVÉS UTILES POUR LE TRAITEMENT DE MALADIES VIRALES

(30) Priority: 25.11.2009 US 264422 P
(43) Date of publication of application: 03.10.2012
(73) Proprietor: Merck Sharp & Dohme Corp., Rahway, NJ 07065-0907 (US)
(72) Inventor: ROSENBLUM, Stuart, B., Kenilworth, New Jersey 07033 (US); CHEN, Kevin, X., Kenilworth, New Jersey 07033 (US); KOZLOWSKI, Joseph, A., Kenilworth, New Jersey 07033 (US); NJOROGE, George F., Kenilworth, New Jersey 07033 (US)
(74) Representative: Böhles, Elena
(86) International application number: PCT/US2010/057708
(87) International publication number: WO 2011/066241

(56) References cited:
- WO-A1-00/20400
- WO-A1-2008/082484
- WO-A1-2008/098368
- WO-A1-2009/102633
- WO-A1-2010/017401
- WO-A1-2010/132538
- US-B1- 7 438 920

## Description

### FIELD OF THE INVENTION

The present invention relates to novel Fused Tricyclic Compounds, compositions comprising at least one Fused Tricyclic Compound, and use of Fused Tricyclic Compounds for treating or preventing a viral infection or a virus-related disorder in a patient.

### BACKGROUND OF THE INVENTION

Hepatitis C virus (HCV) is a major human pathogen, infecting an estimated 170 million persons worldwide. A substantial fraction of these HCV-infected individuals develop serious progressive liver disease, including cirrhosis and hepatocellular carcinoma, which are often fatal. HCV is a (+)-sense single-stranded enveloped RNA virus that has been implicated as the major causative agent in non-A, non-B hepatitis (NANBH), particularly in blood-associated NANBH (BB-NANBH) (see, International Publication No. WO 89/04669 and European Patent Publication No. EP 381 216). NANBH is to be distinguished from other types of viral-induced liver disease, such as hepatitis A virus (HAV), hepatitis B virus (HBV), delta hepatitis virus (HDV), cytomegalovirus (CMV) and Epstein-Barr virus (EBV), as well as from other forms of liver disease such as alcoholism and primary biliar cirrhosis.

The RNA genome of HCV contains a 5'-nontranslated region (5' NTR) of 341 nucleotides, a large open reading frame (ORF) encoding a single polypeptide of 3,010 to 3,040 amino acids, and a 3'-nontranslated region (3'-NTR) of variable length of about 230 nucleotides. HCV is similar in amino acid sequence and genome organization to flaviviruses and pestiviruses, and therefore HCV has been classified as a third genus of the family Flaviviridae.

The 5' NTR, one of the most conserved regions of the viral genome, contains an internal ribosome entry site (IRES) which plays a pivotal role in the initiation of translation of the viral polyprotein. A single long open reading frame encodes a polyprotein, which is co- or post-translationally processed into structural (core, E1, E2 and p7) and nonstructural (NS2, NS3, NS4A, NS4B, NS5A, and NS5B) viral proteins by either cellular or viral proteinases. The 3' NTR consists of three distinct regions: a variable region of about 38 nucleotides following the stop codon of the polyprotein, a polyuridine tract of variable length with interspersed substitutions of cytidines, and 98 nucleotides (nt) at the very 3' end which are highly conserved among various HCV isolates. By analogy to other plus-strand RNA viruses, the 3'-NTR is thought to play an important role in viral RNA synthesis. The order of the genes within the genome is: NH₂-C-E1-E2-p7-NS2-NS3-NS4A-NS4B-NS5A-NS5B-COOH.

More recently, attention has been focused toward the identification of inhibitors of HCV NS5A. HCV NS5A is a 447 amino acid phosphoprotein which lacks a defined enzymatic function. It runs as 56kd and 58kd bands on gels depending on phosphorylation state (Tanji, et al. J. Viol. 69:3980-3986 (1995)). HCV NS5A resides in replication complex and may be responsible for the switch from replication of RNA to production of infectious virus (Huang, Y, et al., Virology 364:1-9 (2007)).

Multicyclic HCV NS5A inhibitors have been reported. U.S. Patent Publication No. US2008/0311075 A1 discloses NS5A inhibitors of formula A: 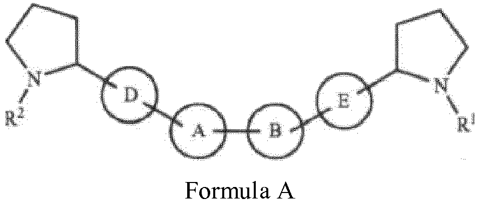 wherein A and B are each phenyl and D and E are each five-membered heteroaromatic rings.

U.S. Patent Publication No. US2008/0044379 A1 discloses NS5A inhibitors of formula B: 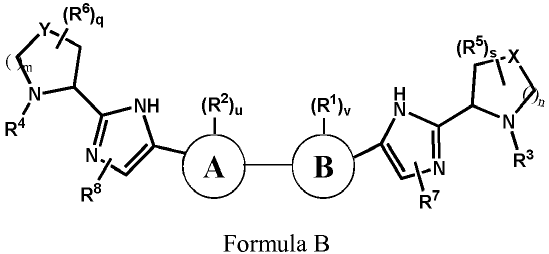 wherein A and B are selected from phenyl and a six-membered heteroaromatic ring.

U.S. Patent Publication No. US2008/0050336 discloses NS5A inhibitors of formula C:

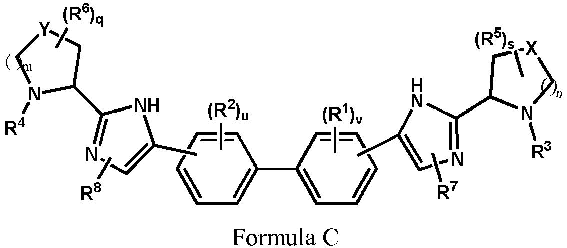

U.S. Patent Publication No. US2008/0044380 A1 discloses NS5A inhibitors of formula D: 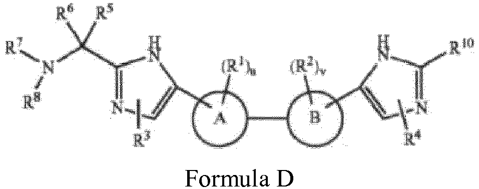 wherein A and B are independently selected from phenyl and a six-membered heteroaromatic ring.

U.S. Patent Publication No. US2009/0202483 discloses hepatitis C virus inhibitors of formula E: 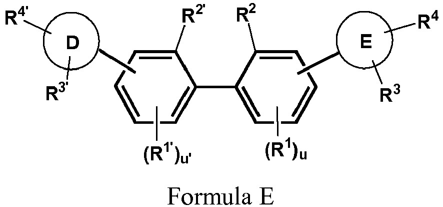 wherein D and E are independently five-membered heteroaromatic rings; R³, R³, R⁴ and R⁴ can each be heterocycloalkyl or substituted amino groups; and wherein R² and R^{2'}, together with the carbon atoms to which they are attached, can join to form a five- to eight-membered unsaturated ring.

U.S. Patent Publication No. US2009/020478 discloses hepatitis C virus inhibitors of formula F: 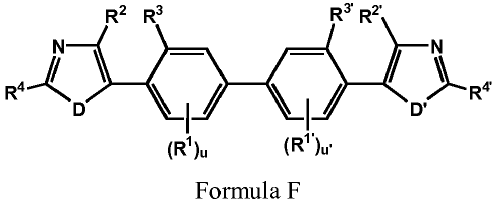 wherein D and D' can each be NH, O or S; R² and R³, together with the carbon atoms to which they are attached, join to form a five- to eight-membered aromatic or non aromatic carbocyclic or heterocyclic ring; R² and R³, together with the carbon atoms to which they are attached, join to form a five- to eight-membered aromatic or non aromatic carbocyclic or heterocyclic ring; and R⁴ and R⁴ are each independently selected from a nitrogen-containing non-aromatic heterocycle or a substituted aminomethyl group.

Other NS5A inhibitors and their use for reducing viral load in HCV infected humans have been described in U.S. Patent Publication No. US2006/0276511.

Despite the intensive effort directed at the treatment and prevention of HCV and related viral infections, there exists a need in the art for non-peptide, small-molecule compounds having desirable or improved physicochemical properties that are useful for inhibiting viruses and treating viral infections and virus-related disorders. Specifically, there exists a need for compounds and methods useful for modulating the activity of serine proteases, including NS3, as well as modulating HCV NS2, NS4A, NS4B, NS5A, NS5B and IRES.

Due to the rapidly mutating rate of HCV and thus the emergence of resistant strains, there exists a need for small molecule compounds which act by different mechanisms that can be combined to treat viral infections and virus-related disorders. This invention addresses that need.

### SUMMARY OF THE INVENTION

In one aspect, the present invention provides Compounds of Formula (I): 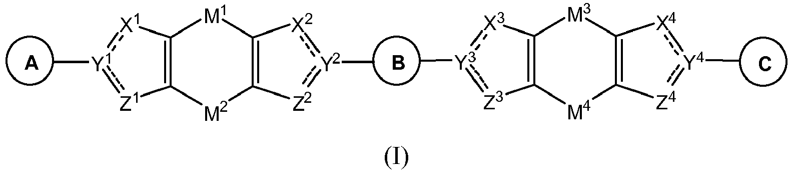 or a pharmaceutically acceptable salt or solvate thereof, wherein the group: 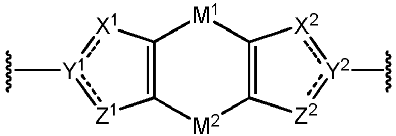 has the structure: 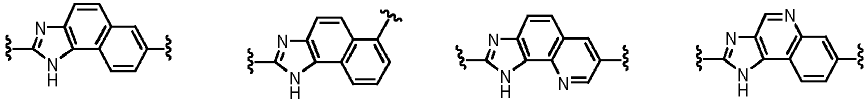 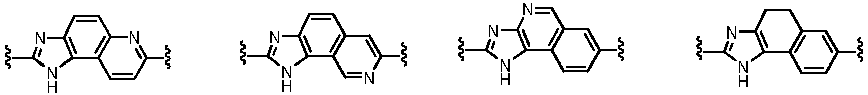 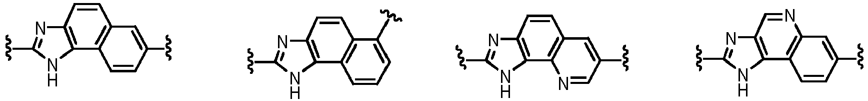 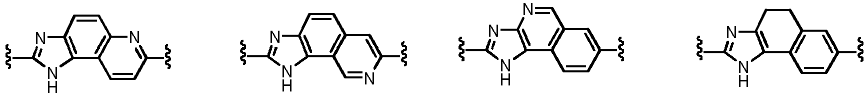 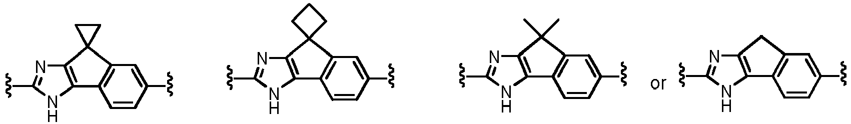 the group: 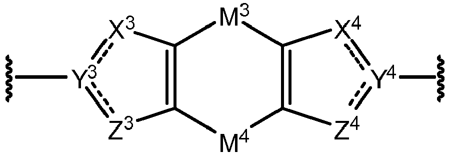 has the structure: 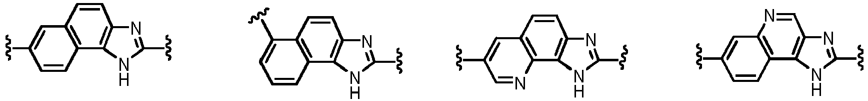 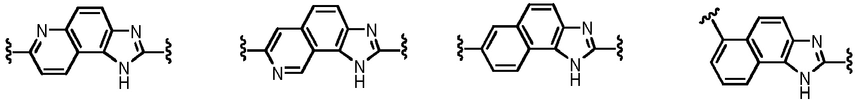 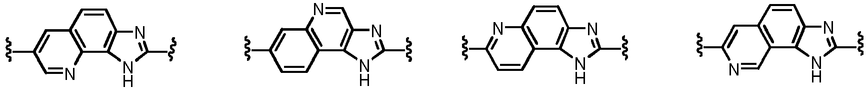 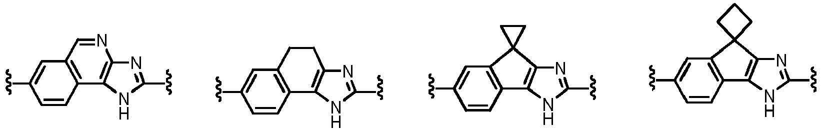 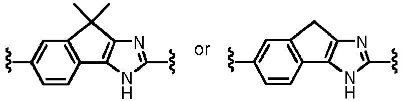 A and C are each: 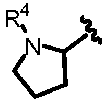 wherein each occurrence of R⁴ is independently: 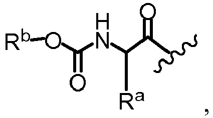 wherein R^{a} is H, alkyl, haloalkyl, cycloalkyl or aryl, and R^{b} is alkyl, haloalkyl or cycloalkyl and
B is a bond or phenylene;
such that at least one of the rings containing: (i) X², Y² and Z² or (ii) X³, Y³ and Z³ is other than a benzene ring.

The Compounds of Formula (I) (also referred to herein as the "Fused Tricyclic Compounds") and pharmaceutically acceptable salts or solvates thereof can be useful for treating or preventing a viral infection or a virus-related disorder in a patient.

The Fused Tricyclic Compounds or pharmaceutically acceptable salts or solvates thereof can also be useful for treating or preventing a viral infection or a virus-related disorder in a patient.

The present invention further provides pharmaceutical compositions comprising an effective amount of at least one Fused Tricyclic Compound or a pharmaceutically acceptable salt or solvate thereof, and a pharmaceutically acceptable carrier. The compositions can be useful for treating or preventing a viral infection or a virus-related disorder in a patient.

The details of the invention are set forth in the accompanying detailed description below.

### DETAILED DESCRIPTION OF THE INVENTION

The present invention provides Fused Tricyclic Compounds, pharmaceutical compositions comprising at least one Fused Tricyclic Compound, and use of the Fused Tricyclic Compounds for treating or preventing a viral infection or a virus-related disorder in a patient.

### Definitions and Abbreviations

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name and an ambiguity exists between the structure and the name, the structure predominates. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc...

As used herein, and throughout this disclosure, the following terms, unless otherwise indicated, shall be understood to have the following meanings:
A "patient" is a human or non-human mammal. In one embodiment, a patient is a human. In another embodiment, a patient is a chimpanzee.

The term "effective amount" as used herein, refers to an amount of Fused Tricyclic Compound and/or an additional therapeutic agent, or a composition thereof that is effective in producing the desired therapeutic, ameliorative, inhibitory or preventative effect when administered to a patient suffering from a viral infection or virus-related disorder. In the combination therapies of the present invention, an effective amount can refer to each individual agent or to the combination as a whole, wherein the amounts of all agents administered are together effective, but wherein the component agent of the combination may not be present individually in an effective amount.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In another embodiment, an alkyl group contains from about 1 to about 6 carbon atoms. Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. An alkyl group may be unsubstituted or substituted by one or more substituents which may be the same or different, each substituent being independently selected from the group consisting of halo, alkenyl, alkynyl, aryl, cycloalkyl, cyano, hydroxy, - O-alkyl, -O-aryl, -alkylene-O-alkyl, alkylthio, -NH₂, -NH(alkyl), -N(alkyl)₂, -NH(cycloalkyl), - O-C(O)-alkyl, -0-C(O)-aryl, -O-C(O)-cycloalkyl, -C(O)OH and -C(O)O-alkyl. In one embodiment, an alkyl group is unsubstituted. In another embodiment, an alkyl group is linear. In another embodiment, an alkyl group is branched. The term "C₁-C₆ alkyl" refers to an alkyl group having from 1 to 6 carbon atoms.

The term "aryl," as used herein, refers to an aromatic monocyclic or multicyclic ring system comprising from about 6 to about 14 carbon atoms. In one embodiment, an aryl group contains from about 6 to about 10 carbon atoms. An aryl group can be optionally substituted with one or more "ring system substituents" which may be the same or different, and are as defined herein below. In one embodiment, an aryl group can be optionally fused to a cycloalkyl or cycloalkanoyl group. Non-limiting examples of aryl groups include phenyl and naphthyl. In one embodiment, an aryl group is unsubstituted. In another embodiment, an aryl group is phenyl.

The term "cycloalkyl," as used herein, refers to a non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 3 to about 7 ring atoms. In another embodiment, a cycloalkyl contains from about 5 to about 6 ring atoms. The term "cycloalkyl" also encompasses a cycloalkyl group, as defined above, which is fused to an aryl (*e.g*., benzene) or heteroaryl ring. In one embodiment, a cycloalkyl group is monocyclic. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl. A cycloalkyl group can be optionally substituted with one or more "ring system substituents" which may be the same or different, and are as defined herein below. In one embodiment, a cycloalkyl group is unsubstituted. The term "3 to 7-membered cycloalkyl" refers to a cycloalkyl group having from 3 to 7 ring carbon atoms. A ring carbon atom of a cycloalkyl group may be functionalized as a carbonyl group. An illustrative example of such a cycloalkyl group (also referred to herein as a "cycloalkanoyl" group) includes, but is not limited to, cyclobutanoyl:

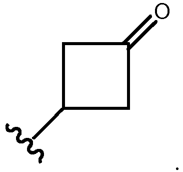

"Halo" means -F, -Cl, -Br or -I. In one embodiment, halo refers to -F, -Cl or -Br.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include -CH₂F, -CHF₂, -CF₃, -CH₂Cl and -CCl₃. The term "C₁-C₆ haloalkyl" refers to a haloalkyl group having from 1 to 6 carbon atoms.

The term "hydroxyalkyl," as used herein, refers to an alkyl group as defined above, wherein one or more of the alkyl group's hydrogen atoms has been replaced with an -OH group. In one embodiment, a hydroxyalkyl group has from 1 to 6 carbon atoms. Non-limiting examples of hydroxyalkyl groups include -CH₂OH, -CH₂CH₂OH, -CH₂CH₂CH₂OH and - CH₂CH(OH)CH₃. The term "C₁-C₆ hydroxyalkyl" refers to a hydroxyalkyl group having from 1 to 6 carbon atoms.

The term "ring system substituent," as used herein, refers to a substituent group attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, - alkylene-aryl, -arylene-alkyl, -alkylene-heteroaryl, -alkenylene-heteroaryl, -alkynyleneheteroaryl, hydroxy, hydroxyalkyl, haloalkyl, -O-alkyl, -O-haloalkyl, -alkylene-O-alkyl, -O-aryl, aralkoxy, acyl, aroyl, halo, nitro, cyano, -SF₅, carboxy, -C(O)O-alkyl, -C(O)O-aryl, -C(O)O-alkylene-aryl, -S(O)-alkyl, -S(O)₂-alkyl, -S(O)-aryl, -S(O)₂-aryl, -S(O)-heteroaryl, -S(O)₂-heteroaryl, -S-alkyl, -S-aryl, -S-heteroaryl, -S-alkylene-aryl, -S-alkylene-heteroaryl, -S(O)₂-alkylene-aryl, -S(O)₂-alkylene-heteroaryl, cycloalkyl, heterocycloalkyl, -O-C(O)-alkyl, -O-C(O)-aryl, -0-C(O)-cycloalkyl, -C(=N-CN)-NH₂, -C(=NH)-NH₂, -C(=NH)-NH(alkyl), Y₁Y₂N-, Y₁Y₂N-alkyl-, Y₁Y₂NC(O)-, and Y₁Y₂NS(O)₂-, wherein Y₁ and Y₂ can be the same or different and are independently selected from the group consisting of hydrogen, alkyl, aryl, cycloalkyl, and -alkylene-aryl. "Ring system substituent" may also mean a single moiety which simultaneously replaces two available hydrogens on two adjacent carbon atoms (one H on each carbon) on a ring system. Examples of such moiety are methylenedioxy, ethylenedioxy, - C(CH₃)₂- and the like which form moieties such as, for example:

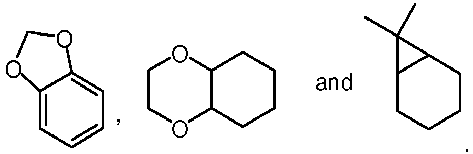

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "purified", "in purified form" or "in isolated and purified form" for a compound refers to the physical state of the compound after being isolated from a synthetic process (*e.g.*, from a reaction mixture), or natural source or combination thereof. Thus, the term "purified", "in purified form" or "in isolated and purified form" for a compound refers to the physical state of the compound after being obtained from a purification process or processes described herein or well-known to the skilled artisan (*e.g*., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Projective Groups in Organic Synthesis (1991), Wiley, New York.

When any variable (*e.g.*, aryl, heterocycle, R², etc.) occurs more than one time in any constituent or in Formula (I), its definition on each occurrence is independent of its definition at every other occurrence.

As used herein, the term "composition" is intended to encompass a product comprising the specified ingredients in the specified amounts, as well as any product which results, directly or indirectly, from combination of the specified ingredients in the specified amounts.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates, and the like. "Hydrate" is a solvate wherein the solvent molecule is H₂O.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTechours., 5(1), article 12 (2004); and A. L. Bingham et al, Chem. Common., 603-604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Fused Tricyclic Compounds can form salts which are also within the scope of this invention. Reference to a Fused Tricyclic Compound herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a Fused Tricyclic Compound contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e.*, non-toxic, physiologically acceptable) salt. In another embodiment, the salt is other than a pharmaceutically acceptable salt. Salts of the Compounds of Formula (I) may be formed, for example, by reacting a Fused Tricyclic Compound with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). These disclosures are incorporated herein by reference thereto.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (*e.g.*, methyl, ethyl, and butyl chlorides, bromides and iodides), dialkyl sulfates (*e.g.*, dimethyl, diethyl, and dibutyl sulfates), long chain halides (*e.g.*, decyl, lauryl, and stearyl chlorides, bromides and iodides), aralkyl halides (*e.g*., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures can be separated into their individual diastereomers on the basis of their physical chemical differences by methods well-known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g*., hydrolyzing) the individual diastereomers to the corresponding pure enantiomers. Sterochemically pure compounds may also be prepared by using chiral starting materials or by employing salt resolution techniques. Also, some of the Fused Tricyclic Compounds may be atropisomers (*e.g*., substituted biaryls) and are considered as part of this invention. Enantiomers can also be directly separated using chiral chromatographic techniques.

It is also possible that the Fused Tricyclic Compounds may exist in different tautomeric forms, and all such forms are embraced within the scope of the invention. For example, all keto-enol and imine-enamine forms of the compounds are included in the invention. It should also be noted that tautomeric forms such as, for example, the moieties: 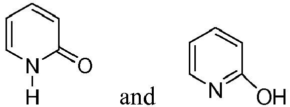 are considered equivalent in certain embodiments of this invention.

All stereoisomers (for example, geometric isomers, optical isomers and the like) of the present compounds (including those of the salts, solvates, hydrates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). If a Fused Tricyclic Compound incorporates a double bond or a fused ring, both the cis- and trans-forms, as well as mixtures, are embraced within the scope of the invention. Also, for example, all keto-enol and imine-enamine forms of the compounds are included in the invention).

Individual stereoisomers of the compounds of the invention may, for example, be substantially free of other isomers, or may be admixed, for example, as racemates or with all other, or other selected, stereoisomers. The chiral centers of the present invention ca have the S or R configuration as defined by the *IUPAC* 1974 Recommendations. The use of the terms "salt", "solvate", "ester", "prodrug" and the like, is intended to apply equally to the salt, solvate, ester and prodrug of enantiomers, stereoisomers, rotamers, tautomers, positional isomers, racemates or prodrugs of the inventive compounds.

The present invention also embraces isotopically-labelled compounds of the present invention which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

Certain isotopically-labelled Fused Tricyclic Compounds (*e.g*., those labeled with ³H and ¹⁴C) are useful in compound and/or substrate tissue distribution assays. In one embodiment, tritiated (*i.e.*, ³H) and carbon-14 (*i.e.*, ¹⁴C) isotopes are employed for their ease of preparation and detectability. In another embodiment, substitution with heavier isotopes such as deuterium (*i.e.*, ²H) may afford certain therapeutic advantages resulting from greater metabolic stability (*e.g*., increased in vivo half-life or reduced dosage requirements). In one embodiment, a Compound of Formula (I) has one or more of its hydrogen atoms replaced with a deuterium atom. Isotopically labelled compounds of Formula (I) can generally be prepared by following procedures analogous to those disclosed in the Schemes and/or in the Examples herein below, by substituting an appropriate isotopically labelled reagent for a non-isotopically labelled reagent.

Polymorphic forms of the Fused Tricyclic Compounds, and of the salts, solvates, hydrates, esters and prodrugs of the Fused Tricyclic Compounds, are intended to be included in the present invention.

Polymorphic forms of the Fused Tricyclic Compounds, and of the salts, solvates, hydrates, esters and prodrugs of the Fused Tricyclic Compounds, are intended to be included in the present invention.

The following abbreviations are used below and have the following meanings: AcOH is acetic acid, Boc is tert-butyloxycarbonyl, Boc-Pro-OH is Boc protected praline, DBU is 1,8-diazabicyclo[5.4.0]undec-7-ene, DCC is dicyclohexylcarbodiimide, DME is dimethoxyethane, DMF is dimethylformamide, DMSO is dimethylsulfoxide, EDCI is 1-(3-dimethylaminopropyl)-3-ethylcarbodiimide, EtOAc is ethyl acetate, EtOH is ethanol, HATU is is O-(7-azabenzotriazol-1-yl)-N,N,N',N'-tetramethyluronium hexafluorophosphate, HOBt is 1-hydroxybenzotriazole, HPLC is high performance liquid chromatography, KOAc is potassium acetate, LRMS is low resolution mass spectrometry, MeOH is methanol, MTBE is PyBrop is bromotripyrrolidinophosphonium hexafluorophosphate, TFA is trifluoroacetic acid and TLC is thin-layer chromatography.

### The Compounds of Formula (I)

The present invention provides Fused Tricyclic Compounds of Formula (I): 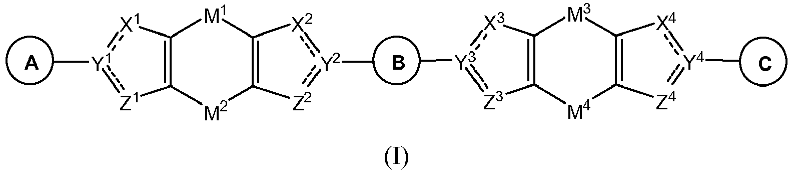 and pharmaceutically acceptable salts, solvates, prodrugs and esters thereof, wherein A, B, C, M¹,M²,M³,M⁴,X¹,X²,X³,X⁴,Y¹,Y²,Y³,Y²,Z¹,Z²,Z³ and Z⁴ are defined above for the Compounds of Formula (I).

In one embodiment, A is 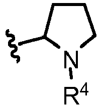 and R⁴ is: 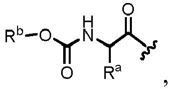 wherein R^{a} is H, alkyl, haloalkyl, cycloalkyl or aryl, and R^{b} is alkyl, haloalkyl or cycloalkyl.

In another embodiment, A is: 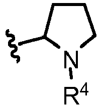 and R⁴ is: 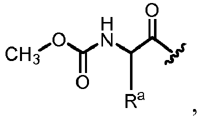 wherein R^{a} is H, methyl, ethyl, propyl, isopropyl, -CH(CH₃)(OCH₃), t-butyl, cyclopropyl, -CH₂CH₂CF₃ or phenyl.

In another embodiment, A is: 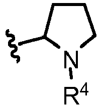 and R⁴ is:

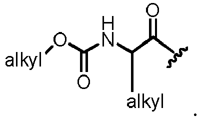

In yet another embodiment, A is: 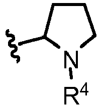 and R⁴ is

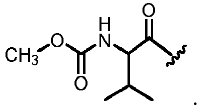

In one embodiment, B is a bond.

In another embodiment, B is phenylene.

In another embodiment, B is:

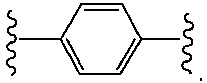

In another embodiment, C is 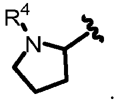 and R⁴ is: 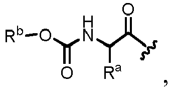 wherein R^{a} is H, alkyl, haloalkyl, cycloalkyl or aryl, and R^{b} is alkyl, haloalkyl or cycloalkyl.

In another embodiment, C is: 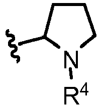 and R⁴ is: 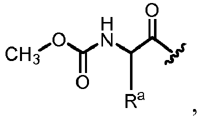 wherein R^{a} is H, methyl, ethyl, propyl, isopropyl, -CH(CH₃)(OCH₃), t-butyl, cyclopropyl, -CH₂CH₂CF₃ or phenyl.

In another embodiment, C is: 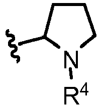 and R⁴ is:

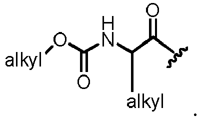

In yet another embodiment, C is: 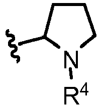 and R⁴ is

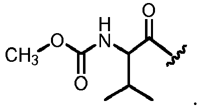

In another embodiment, A and C are each independently: 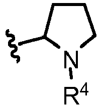 and each R⁴ is independently 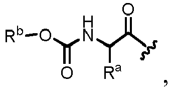 wherein R^{a} is H, alkyl, haloalkyl, cycloalkyl or aryl, and R^{b} is alkyl, haloalkyl or cycloalkyl.

In another embodiment, A and C are each independently 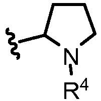 each R⁴ is independently: 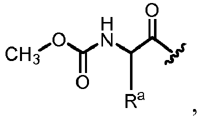 wherein R^{a} is H, methyl, ethyl, propyl, isopropyl, -CH(CH₃)(OCH₃), t-butyl, cyclopropyl, -CH₂CH₂CF₃ or phenyl.

In another embodiment, A and C are each independently 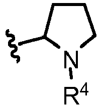 each R⁴ is independently:

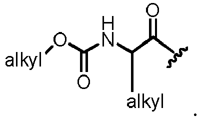

In one embodiment, A and C are each independently selected from: 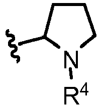 and each occurrence of R⁴ is independently selected from:

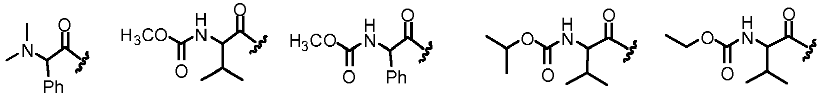

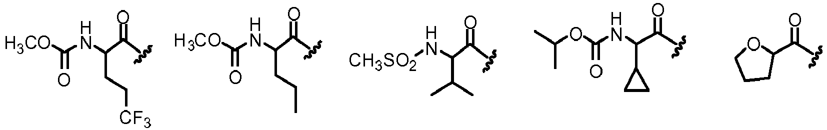

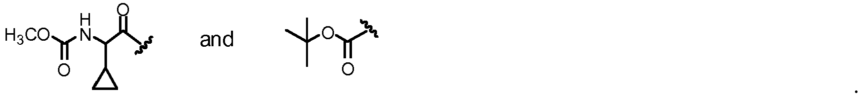

In another embodiment, A and C are each independently 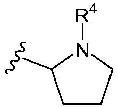 and each occurrence of R⁴ is independently selected from:

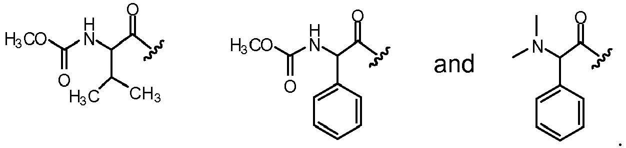

In still another embodiment, A and C are each independently: 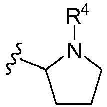 and each occurrence of R⁴ is:

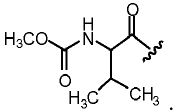

In one embodiment, the group: 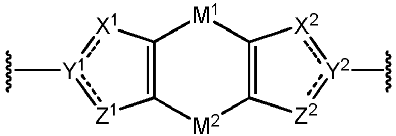 has the structure:

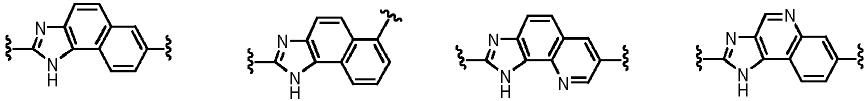

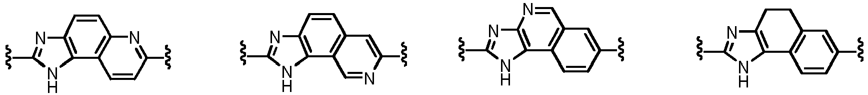

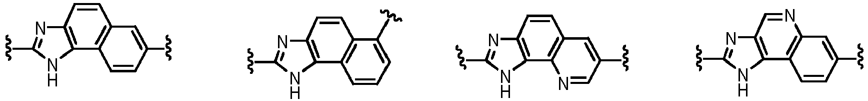

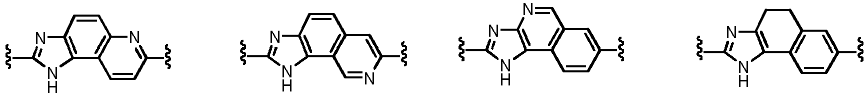

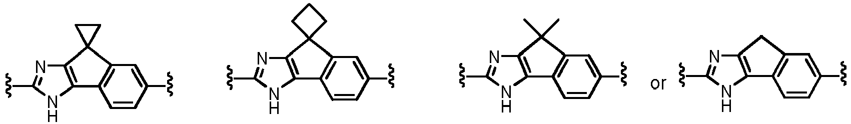

In another embodiment, the group: 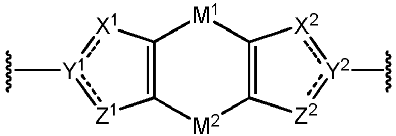 has the structure:

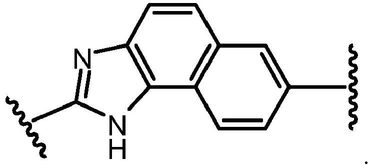

In one embodiment, the group: 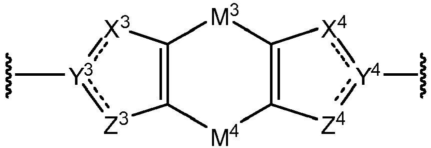 has the structure: 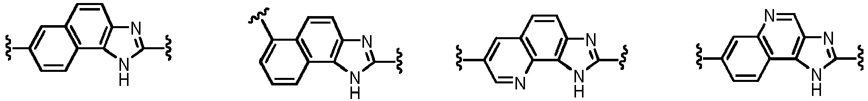 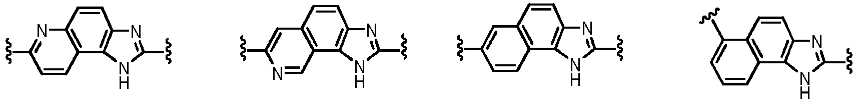 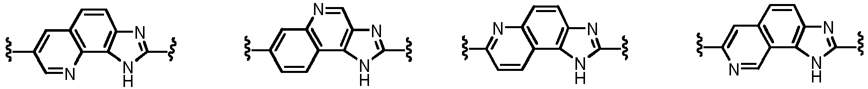 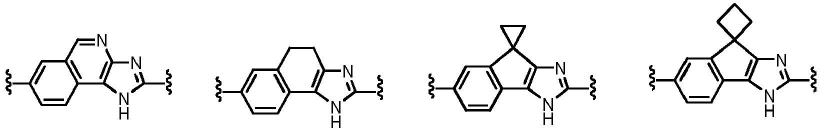 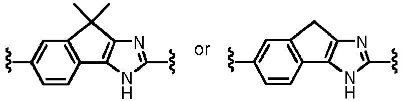 wherein either available bond on any of the above divalent groups can connect to either group flanking the above divalent groups.

In another embodiment, the group: 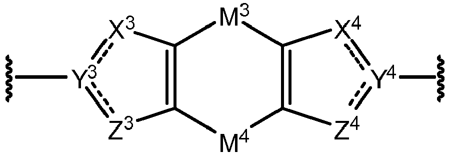 has the structure:

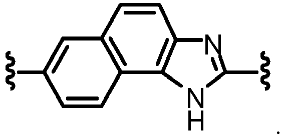

In one embodiment, B is a bond; the group: 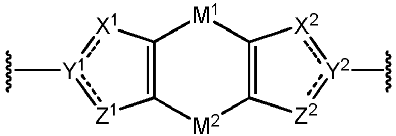 has the structure: 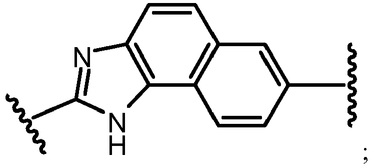 and the group: 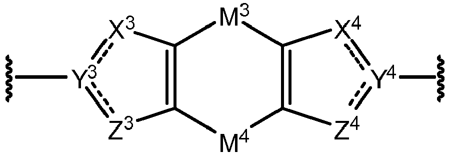 has the structure:

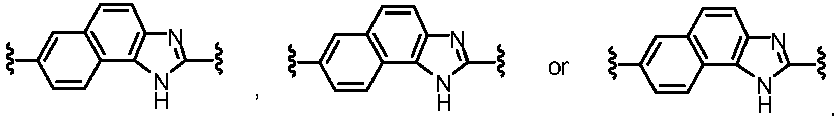

In one embodiment, each occurrence of R⁴ is independently selected from:

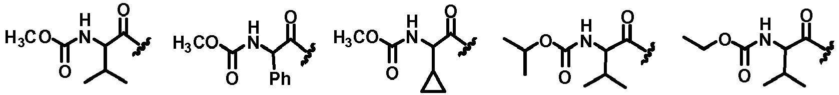

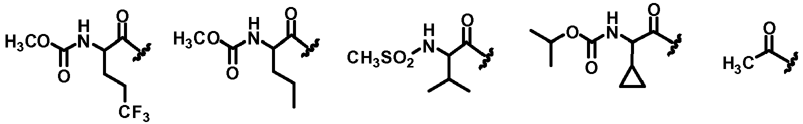

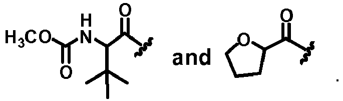

In another embodiment, each occurrence of R⁴ is independently: 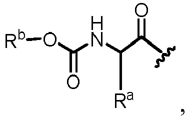 wherein R^{a} is H, alkyl, haloalkyl, cycloalkyl or aryl, and R^{b} is alkyl, haloalkyl or cycloalkyl.

In another embodiment, each occurrence of R⁴ is independently: 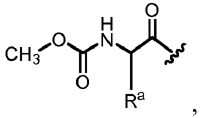 wherein R^{a} is H, methyl, ethyl, propyl, isopropyl, -CH(CH₃)(OCH₃), t-butyl, cyclopropyl, -CH₂CH₂CF₃ or phenyl.

In another embodiment, each occurrence of R⁴ is independently:

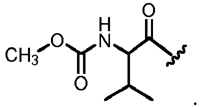

In one embodiment, A and C are each 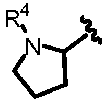 and B is a bond.

In a further embodiment, A and C are each 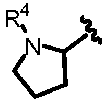 and each occurrence of R⁴ is independently selected from:

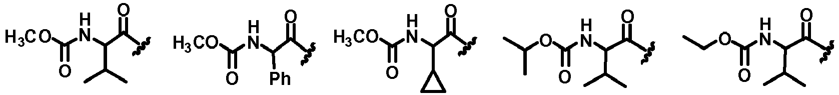

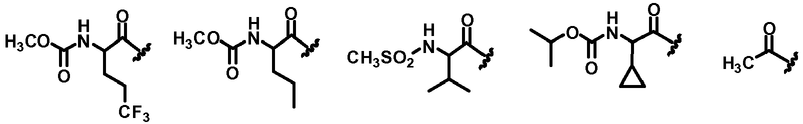

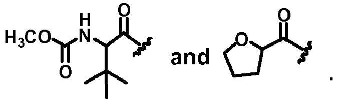

In a further embodiment, A and C are each 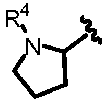 and each occurrence of R⁴ is independently selected from:

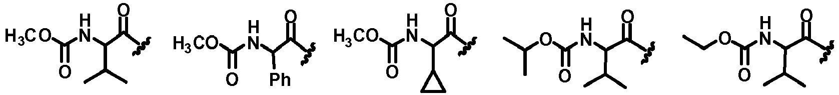

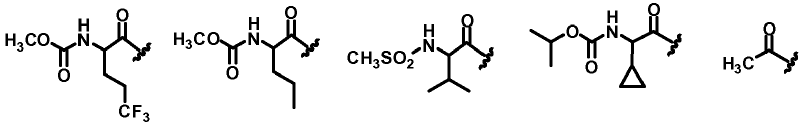

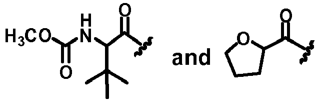

In one embodiment, A and C are each 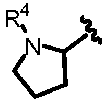 and each occurrence of R⁴ is independently: 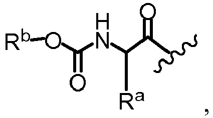 wherein R^{a} is H, alkyl, haloalkyl, cycloalkyl or aryl, and R^{b} is alkyl, haloalkyl or cycloalkyl.

In another embodiment, A and C are each 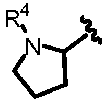 and each occurrence of R⁴ is independently: 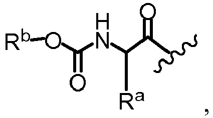 wherein R^{a} is H, methyl, ethyl, propyl, isopropyl, -CH(CH₃)(OCH₃), t-butyl, cyclopropyl, -CH₂CH₂CF₃ or phenyl, and R^{b} is methyl, ethyl, propyl, isopropyl, t-butyl, cyclopropyl, or -CH₂CH₂CF₃.

In another embodiment, A and C are each 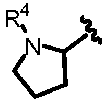 and each occurrence of R⁴ is:

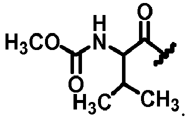

In still another embodiment, the group: 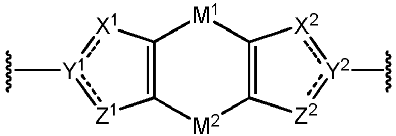 has the structure: 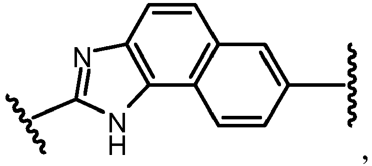 and the group: 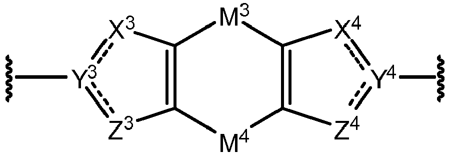 has the structure:

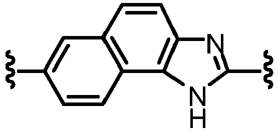

In one embodiment A, B, C, M¹, M², M³, M⁴, X¹, X², X³, X⁴, Y¹, Y², Y³, Y², Z¹, Z², Z³ and Z⁴ in the Compounds of Formula (I) are selected independently from each other.

In another embodiment, a Compound of Formula (I) is in purified form.

In another embodiment, a Compound of Formula (I) has one or more of its hydrogen atoms replaced with a deuterium atom.

Non-limiting examples of the Compounds of Formula (I) include compounds 1-69 as set forth below: 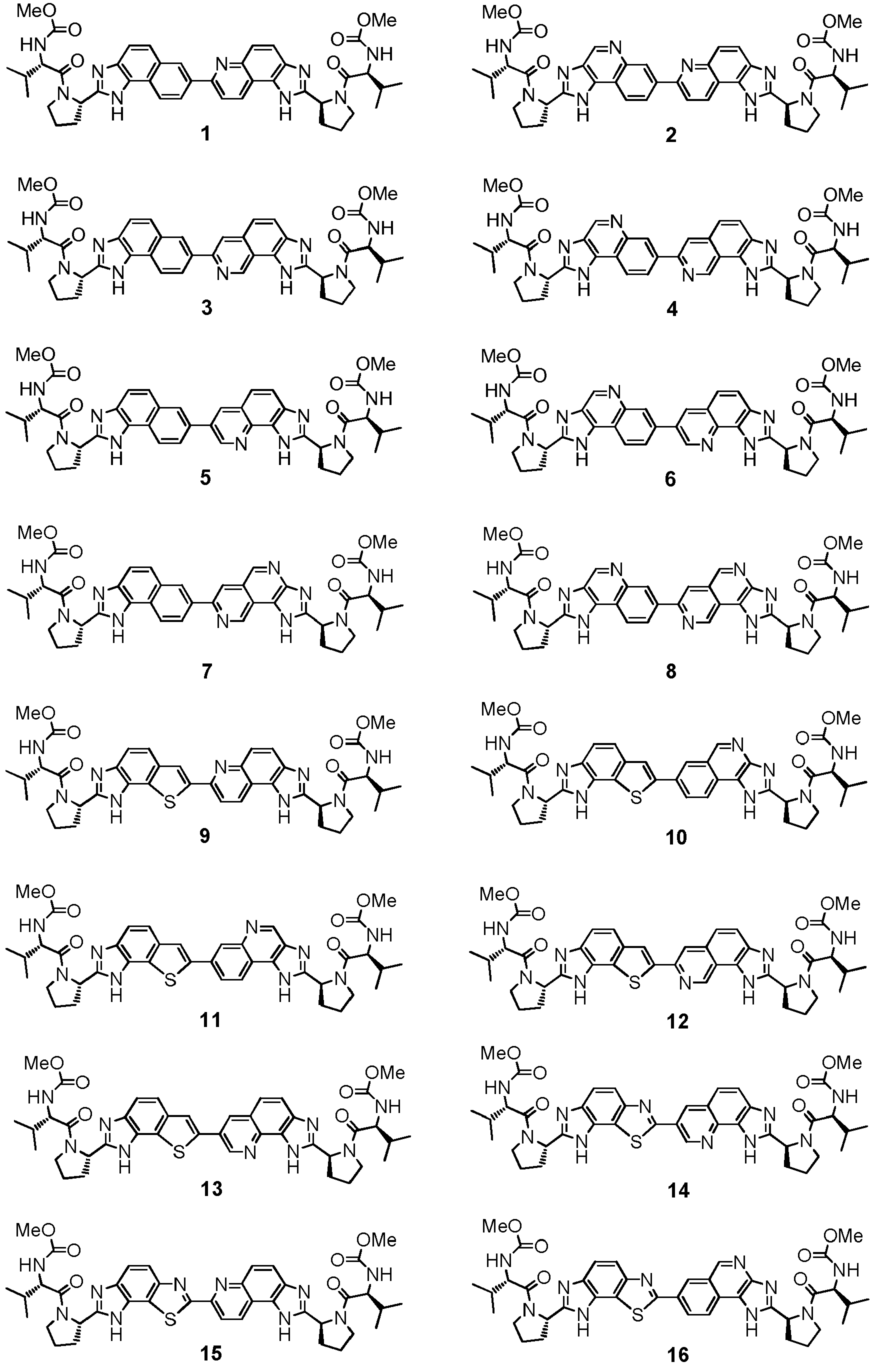 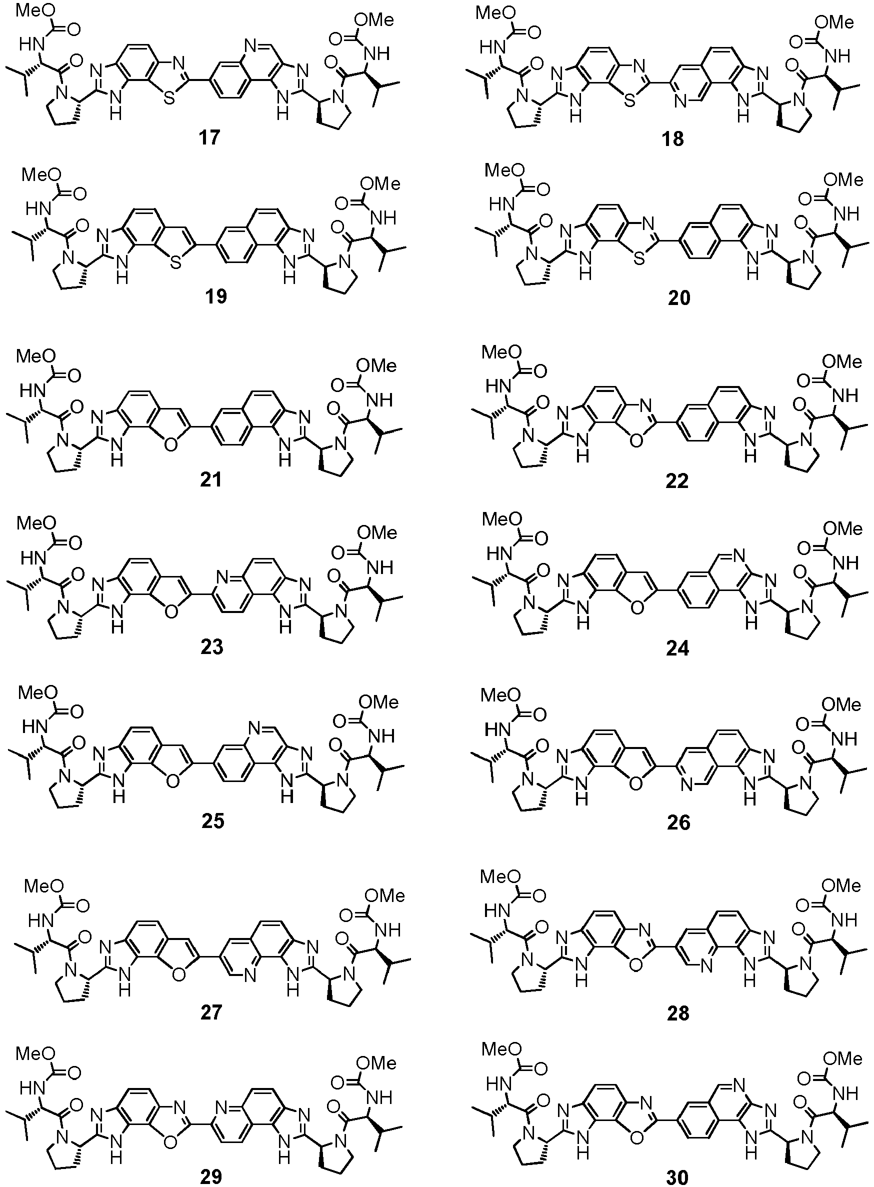 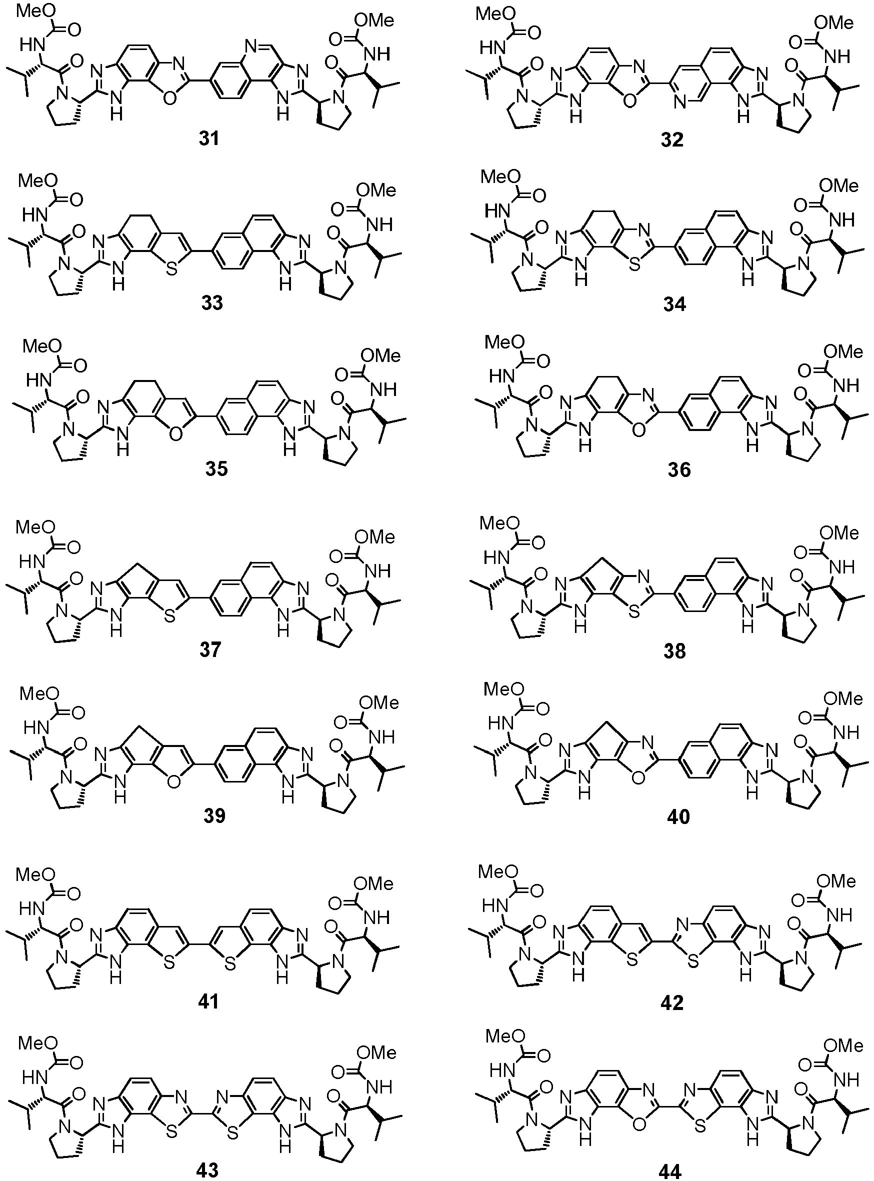 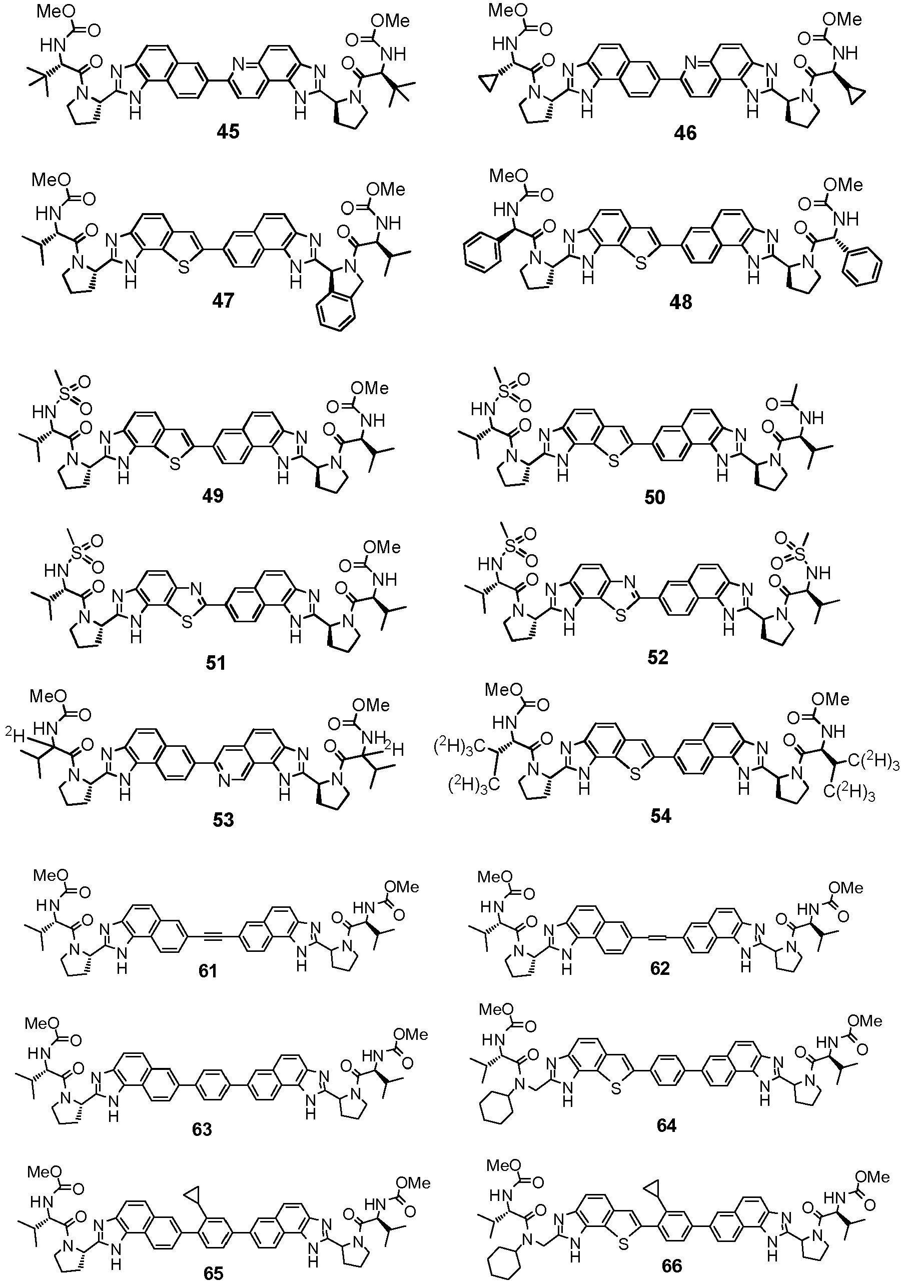 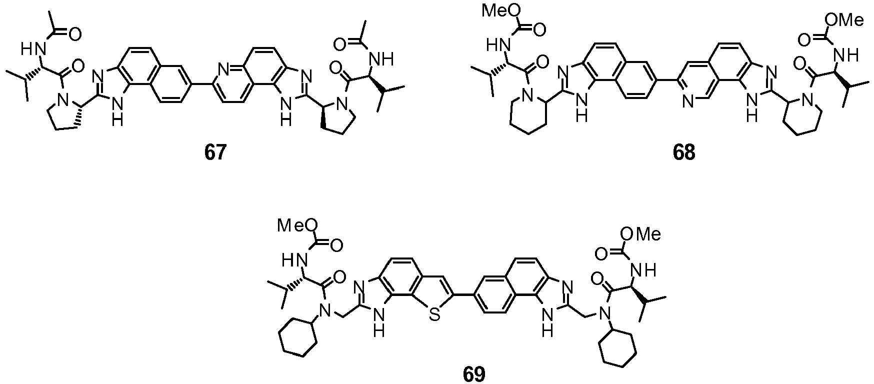 and pharmaceutically acceptable salts or solvates thereof.

### Methods For Making the Compounds of Formula (I)

The Compounds of Formula (I) may be prepared from known or readily prepared starting materials, following methods known to one skilled in the art of organic synthesis. Methods useful for making the Compounds of Formula (I) are set forth in the Examples below and generalized below in Schemes 1-5. Alternative synthetic pathways and analogous structures will be apparent to those skilled in the art of organic synthesis. All stereoisomers and tautomeric forms of the compounds are contemplated.

Some commercially available starting materials and intermediates used for the synthesis of the Compounds of Formula (I) are available which contain intact fused tricyclic tricyclic ring systems. These starting materials and intermediates are available from commercial suppliers such as Sigma-Aldrich (St. Louis, MO) and Acros Organics Co. (Fair Lawn, NJ). Such starting materials and intermediates compounds are used as received. When such fused tricyclic moieties are not commercially available, they can be prepared using methods well-known to those skilled in the art of organic synthesis. Such synthetic methods include, but are not limited to, those described in Kricka et al., J. Chem. Soc. Perkin Trans I, 859-863 (1973); Kricka et al., Chem. Rew., 74, 101-123, (1974); Kurfuerst et al., Coll. Czech. Chem. Comm., 54, 1705-1715, (1989); Saroja et al., J. Org. Chem. 69, 987-990, (2004); Fanta et al., Synth. 9-21, (1974), U.S. Patent Publication No. US2005038037; and International Publication No. WO2004039859.

Scheme 1 shows a method useful for making the naphtyl imidazole compounds of formula **A6** and **A7**, which are useful intermediates for making the Compounds of Formula (I). 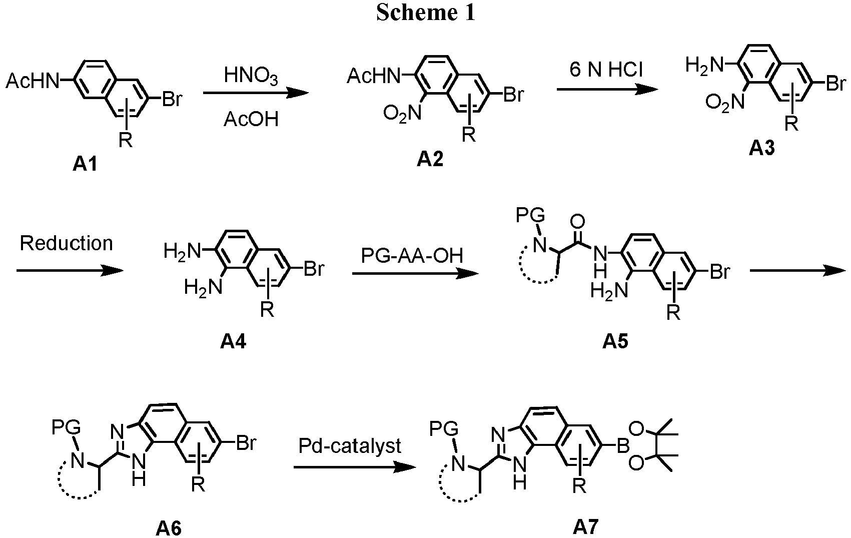 wherein PG-AA-OH refers to an amino acid that has its N-terminus protected by a protecting group (PG), such as Boc.

Nitration of bromonaphthal acetamide **A1** provides nitro analog **A2** (J. Am. Chim. Soc, 73:4297 (1997)). The removal of acetyl group under acidic conditions followed by reduction of the nitro group should afford diaminonaphthalene **A4**. Coupling of the aniline to an amino acid gives an amide, which upon heating in acetic acid will cyclize to provide tricyclic bormonaphthalimidazole **A6**. The bromide could be converted to a boronate **A7** with a palladium catalyst.

Scheme 2 shows a method useful for making the quinolineimidazole compounds of formula **B6**, which are useful intermediates for making the Compounds of Formula (I).

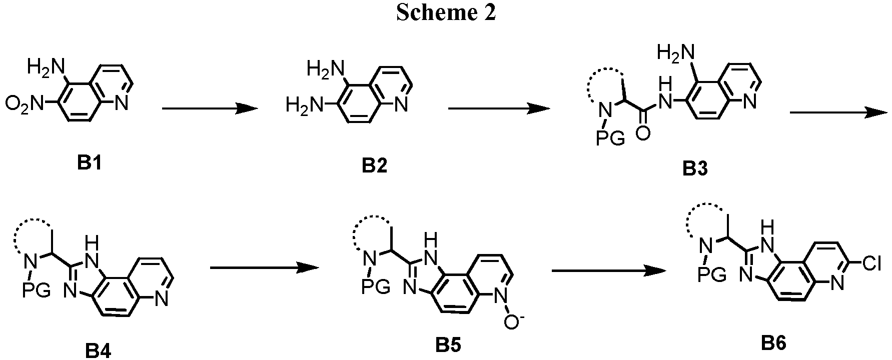

Commercially available aminonitroquinoline **B1** can be reduced to diaminoquinoline **B2**, which is then coupled to an amino acid to provide an amide **B3**. It can then be cyclized to quinolineimidazole **B4** under acidic conditions. *N*-oxide **B5** can then be obtained with *m-*chloroperbenzoic acid. Upon treatment with phosphorous oxychloride, **B5** should give the desired chloroquinoline **B6**, which can used in Suzuki coupling reactions.

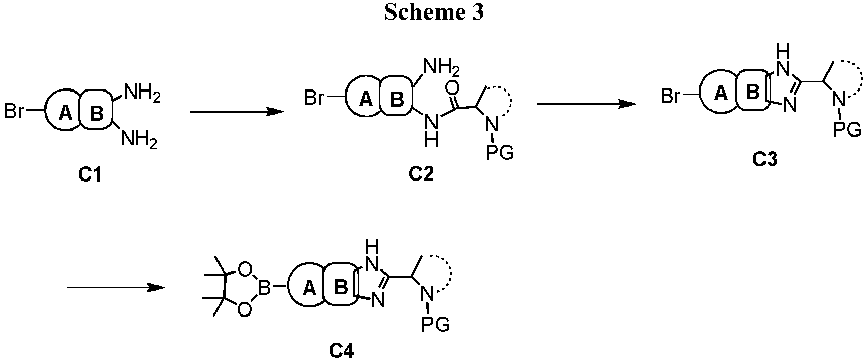

Similar to **A4** and **B2**, an aromatic diamine **C1** could be converted to a bicyclic imidazole **C3** through a two step coupling-cyclization procedure (Scheme 3). The corresponding boronate **C4** can then be obtained from bromide **C3**. Both **C3** and **C4** can be used in a Suzuki coupling to provide the precursors for the final target molecules.

Scheme 4 shows methods useful for making the Compounds of Formula (I) via a Suzuki Coupling process.

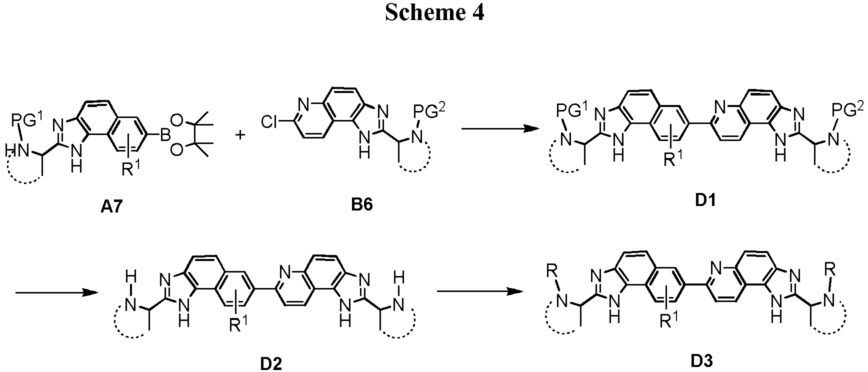

With the advanced coupling partners on hand, the Suzuki coupling between an aryl halide (e.g., **A6** and **B6**) and an aryl boronate (e.g., **A7**) should provide biaryl products such as **D1** (Scheme 4) using, for example the methods disclosed in Angew Chem. Int. Ed. Engl., 40, 4544 (2001). After removal of the protecting groups at nitrogen at both ends, an appropriate cap can be introduced to the resulted bis-amine using reactions including, but not limited to acylation (with an acyl chloride or amino acid coupling reagent such as HATU or HOBt/EDCI), sulfonylation (with a sulfonyl chloride) or alkylation (with alkyl halide or reductive amination) to provide the desired compounds of type **D3**.

Scheme 5 shows methods useful for making the Compounds of Formula (I) via a Suzuki coupling process.

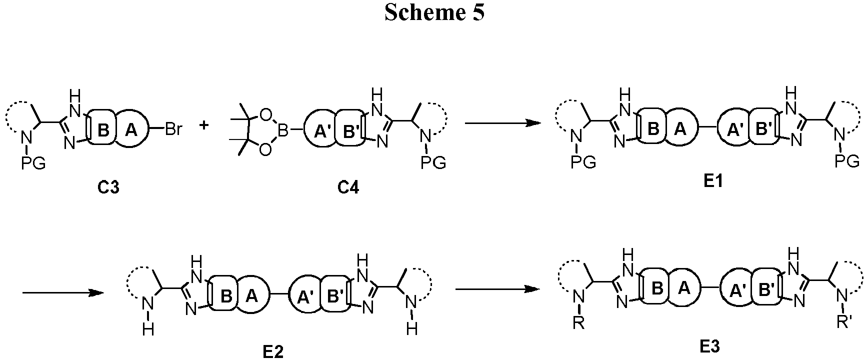

A Suzuki coupling between protected fused tricyclic boronate **C4** (or boronic acid, not shown) and the fused tricyclic aryl halide (such as bromide **C3**) using the methods similar to, but not limited to those reported in Angew Chem. Int. Ed. Engl., 40, 4544 (2001) should give compound of type **E1**, wherein at least one of the ring **A** or **A'** is a 5- or 6-membered heterocycle. The product **E1** is processed to the final targets **E3** after removal the protecting groups and introducing the capping groups on the two amines using the method similar to the preparation of **D3**.

In some of bicyclic and fused tricyclic compounds contemplated in Scheme 1-5, the amino acids (such as, but not limited to proline, 4,4-difluoroproline, (S)-2-piperidine carboxylic acid, valine, alanine, norvaline, etc.) are incorporated as part of structures. Methods have been described in the general literature as well as in Banchard US 2009/0068140 (Published March 9th 2009) for the preparation of such amino acid-derived intermediates.

One skilled in the art of organic synthesis will recognize that the synthesis of fused tricyclic cores in Formula (I) may require protection of certain functional groups (*i.e.*, derivatization for the purpose of chemical compatibility with a particular reaction condition). Suitable protecting groups for the various functional groups of these compounds and methods for their installation and removal can be found in Greene et al., Projective Groups in Organic Synthesis, Wiley-Interscience, New York, (1999).

One skilled in the art of organic synthesis will also recognize that one route for the synthesis of fused bi-aryl tricyclic cores in Formula (I) may be more desirable depending on the choice of appendage substituents. Additionally, one skilled in the art will recognize that in some cases the order of reactions may differ from that presented herein to avoid functional group incompatibilities and can amend the synthetic route accordingly.

One skilled in the art of organic synthesis will recognize that the synthesis of certain fused tricyclic cores in Formula (I) require the construction of an amide bond. Methods useful for making such amide bonds, include but are not limited to, the use of a reactive carboxy derivative (*e.g*., an acid halide, or ester at elevated temperatures) or the use of an acid with a coupling reagent (*e.g*., HOBt, EDCI, DCC, HATU, PyBrop) with an amine.

The preparation of ring systems contemplated in this invention have been described in the literature and in compendia such as "Comprehensive Heterocyclic Chemistry" editions I, II and III, published by Elsevier and edited by A.R. Katritzky & R JK Taylor. Manipulation of the required substitution patterns have also been described in the available chemical literature as summarized in compendia such as "Comprehensive Organic Chemistry" published by Elsevier and edited by DH R. Barton and W. D. Ollis; "Comprehensive Organic Functional Group Transformations" edited by edited by A.R. Katritzky & R JK Taylor and "Comprehensive Organic Transformation" published by Wily-CVH and edited by R. C. Larock.

The starting materials used and the intermediates prepared using the methods set forth in the Schemes above may be isolated and purified if desired using conventional techniques, including but not limited to filtration, distillation, crystallization, chromatography and alike. Such materials can be characterized using conventional means, including physical constants and spectral data.

### EXAMPLES

### General Methods

Solvents, reagents, and intermediates that are commercially available were used as received. Reagents and intermediates that are not commercially available were prepared in the manner as described below. ¹H NMR spectra were obtained on a Bruker Avance 500 (500 MHz) and are reported as ppm down field from Me₄Si with number of protons, multiplicities, and coupling constants in Hertz indicated parenthetically. Where LC/MS data are presented, analyses was performed using an Applied Bio systems API-100 mass spectrometer and Shimadzu SCL-10A LC column: Altech platinum C18, 3 micron, 33 mm x 7mm ID; gradient flow: 0 min - 10% CH₃CN, 5 min - 95% CH₃CN, 5-7 min - 95% CH₃CN, 7 min - stop. The observed parent ions are given. Flash column chromatography was performed using pre-packed normal phase silica from Biotage, Inc. or bulk silica from Fisher Scientific. Unless otherwise indicated, column chromatography was performed using a gradient elution of hexanes/ethyl acetate, from 100% hexanes to 100% ethyl acetate.

### Example 1

### Preparation of Intermediate Compound 1G

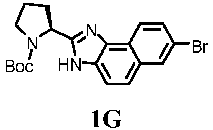

### Step A - Preparation of Compound 1A

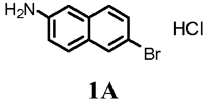

A mixture of 6-bromo-2-naphthoic acid (80.3 g, 319 mmol), diphenylphosphorylazide (71 mL, 352 mmol) and triethylamine (50 mL, 358 mmol) in *tert*-butanol (400 mL) was heated to reflux for 15 hours then cooled to room temperature. The reaction mixture was poured over saturated NaHCO₃ (600 mL) and stirred vigorously for 30 minutes. The solids were filtered, washed with water (200 mL) and dried under vacuum at 65 °C. The resulting white solid was suspended in MeOH (500 mL), then HCl(g) was bubbled into the mixture at -78 °C until saturated. The reaction mixture was stirred at room temperature for 15 hours, then the resulting solids were collected by filtration, and washed with ice-cold MeOH (100 mL) to provide Compound **1A** as an off-white solid (74.8 g, 91%). ¹H NMR (DMSO-*d*₆) δ 10.5-10.0 (br s, 3H), 8.23 (s, 1H), 7.99 (d, *J* = 9.0 Hz, 1H), 7.92 (d, *J* = 9.0 Hz, 1H), 7.84 (s, 1H), 7.68-7.65 (m, 1H), 7.56-7.51 (m, 1H). LRMS: (M+2H)⁺ = 223.

### Step B - Reparation of Compound 1B

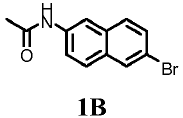

To the solution of Compound **1A** (74.8 g, 289 mmol) and triethylamine (120 mL, 860 mmol) in CH₂Cl₂ (500 mL) at 0 °C was added acetic anhydride (27.5 mL, 292 mmol). The reaction mixture was warmed to room temperature and allowed to stir at this temperature for 1.5 hours. The mixture was filtered and the filtrate concentrated *in vacuo*. The resulting residue was triturated with hexanes (500 mL) and the solids were filtered, washed with hexanes (100 mL) and dried *in vacuo* at 55 °C to provide Compound **1B** as an off-white solid (60.6 g, 79%). ¹H NMR (DMSO-*d*₆) δ 10.1 (s, 1H), 8.30 (s, 1H), 8.09 (s, 1H), 7.85-7.76 (m, 2H), 7.62-7.53 (m, 2H), 2.10 (s, 3H). LRMS: (M+H)⁺ = 265.

### Step C - Reparation of Compound 1C

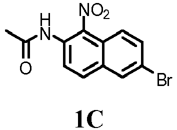

To a solution of compound **1B** (60.6 g, 229 mmol) and acetic anhydride (120 mL) in acetic acid (500 mL) at 0°C was added a solution of fuming nitric acid (36 mL) in AcOH (84 mL) dropwise over 2 hours. The resulting mixture was warmed to room temperature and stirred vigorously for 4.5 hours. The reaction mixture was filtered and the collected solids were washed with water (100 mL). The washed solids were recrystallized from EtOH (1.4 L) to provide Compound **1C** as an off-white solid (58.5 g, 83%). ¹H NMR (DMSO-*d*₆) δ 8.95 (br s, 1H), 8.46 (d, *J* = 9.0 Hz, 1H), 8.00 (s, 1H), 7.92-7.87 (m, 2H), 7.72-7.67 (m, 1H), 2.28 (s, 3H).

### Step D - Reparation of Compound 1D

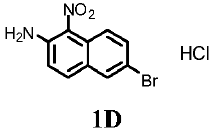

To a solution of Compound **1C** (58.5 g, 189 mmol) in MeOH (150 mL) was added 6 N HCl (150 mL) and the mixture was heated to 75 °C and allowed to stir at this temperature for 6 hours, then cooled to room temperature. The resulting solids were filtered, rinsed with water (100 mL) and dried *in vacuo* at 55 °C to provide Compound **1D** as a yellow solid (47.9 g, 95%). ¹H NMR (DMSO-*d*₆) δ 8.45 (d, *J* = 9.6 Hz, 1H), 8.09-8.00 (m, 3H), 7.84 (d, *J* = 9.6 Hz, 1H), 7.73-7.67 (m, 1H), 7.21 (d, *J* = 9.6 Hz, 1H), 3.33 (br s, 1H).

### Step E - Preparation of Compound 1E

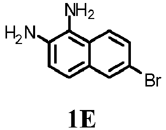

To a solution of Compound **1D** (47.9 g, 179 mmol) and ammonium chloride (14.4 g, 269 mmol) in water (100 mL) and THF (250 mL) was added iron powder (50 g, 895 mmol). The resulting mixture was heated to 60 °C and allowed to stir vigorously at this temperature for 3 hours, then cooled to room temperature. The reaction mixture was filtered over Celite® and rinsed with MeOH until the Celite® was colorless. The filtrate was concentrated *in vacuo* and purified immediately on a silica gel plug (18 cm L × 14 cm W) eluting with 1% MeOH/CH₂Cl₂ (7 L) to provide Compound **1E** as a crude brown solid (40.5 g, 95%). ¹H NMR (DMSO-*d*₆) δ 7.85-7.79 (m, 2H), 7.32-7.29 (m, 1H), 7.03-6.96 (m, 2H), 4.86 (br s, 4H). LRMS: (M+H)⁺ = 238.

### Step F - Reparation of Compound 1F

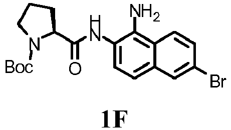

To a solution of compound **1E** (40.5 g, 171 mmol), N-Boc-proline (45.0 g, 209 mmol) and N,N-diisopropylethylamine (90 mL, 517 mmol) in anhydrous DMF (1 L) at 0 °C was added HATU (78 g, 205 mmol). The resulting mixture was warmed to room temperature then stirred at this temperature for 9 hours. Water (1.5 L) was added to the reaction mixture and the resulting aqueous solution was extracted with MTBE (3 × 1.5 L). The combined organics were washed with brine (3 × 1L), dried over Na₂SO₄, filtered and concentrated *in vacuo.* The resulting residue was dissolved in MeOH (75 mL) and water (1.5 L) was added. The resulting heterogeneous mixture was stirred vigorously for 2 hours and filtered. The filter cake was washed with water (1 L) and dried *in vacuo* at 55 °C to provide Compound **1F** as an off-white solid (66.5 g, 90%). ¹H NMR (DMSO-*d*₆) δ 9.45-9.42 (m, 1H), 8.12-8.09 (m, 1H), 8.00 (s, 1H), 7.52-7.47 (m, 1H), 7.36-7.33 (m, 1H), 7.19-7.08 (m, 1H), 5.58 (s, 1H), 5.45 (s, 1H), 4.35-4.21 (m, 1H), 3.45-3.31 (m, 2H), 2.33-2.13 (m, 1H), 2.0-1.75 (m, 3H), 1.46-1.38 (m, 9H).

### Step G - Preparation of Compound 1G

A solution of compound **1F** (66.5 g, 153 mmol) and AcOH (500 mL) was heated to 60 °C and allowed to stir at this temperature for 1 hour. After cooling to room temperature, water (1 L) was added and the resulting solution was adjusted to pH 8 using solid sodium carbonate. The resulting mixture was extracted with CH₂Cl₂ (2 × 1L), dried over Na₂SO₄, filtered and concentrated *in vacuo* to provide Compound **1G** as a crude brown solid (63.7 g, quant.), which was used without further purification. ¹H NMR (DMSO-*d*₆) δ 13.0-12.5 (m, 1H), 8.34 (d, *J* = 9.0 Hz, 1H), 8.25-8.23 (m, 1H), 7.78-7.60 (m, 3H), 5.11-4.93 (m, 1H), 3.70-3.56 (m, 1H), 3.51-3.39 (m, 1H), 2.45-2.24 (m, 1H), 2.13-1.85 (m, 3H), 1.49-0.95 (m, 9H). LRMS: (M+H)⁺ = 416.

### Example 2

### Preparation of Intermediate Compound 2A

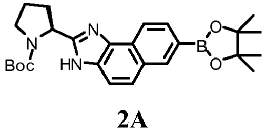

To a solution of compound **1G** (21 g, 50.4 mmol), bis(pinacolato) diboron (14.1 g, 55.5 mmol), and KOAc (7.5 g, 76.4 mmol) in 1,4-dioxane (20 mL) was added a premixed solution of Pd(dba)₂ (1.16 g, 2.01 mmol) and tricyclohexylphosphine (1.14 g, 4.06 mmol) in 1,4-dioxane (10 mL). The resulting mixture was heated to 100 °C and allowed to stir at this temperature for 4 hours then cooled to room temperature. The reaction mixture was filtered through Celite®, the pad was washed with CH₂Cl₂ (100 mL) and the filtrate concentrated *in vacuo*. The residue obtained was purified using flash chromatography on an ISCO 330 g Redi-Sep column using a gradient of 0-70% EtOAc/hexanes as eluent to provide Compound **2A** as a yellow solid (19 g, 82%). ¹H NMR (DMSO-*d*₆) δ 13.0-12.5 (m, 1H), 8.40-8.36 (m, 2H), 7.84-7.63 (m, 3H), 5.13-4.93 (m, 1H), 3.73-3.57 (m, 1H), 3.51-3.41 (m, 1H), 2.44-2.25 (m, 1H), 2.18-1.95 (m, 3H), 1.40-1.02 (m, 21H). LRMS: (M+H)⁺ = 464.

### Example 3

### Preparation of Intermediate Compound 3E

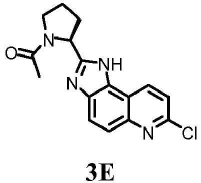

### Step A: Preparation of Compound 3A

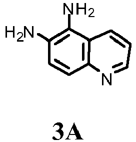

To a solution of 50% palladium on carbon (10% wet, 250 mg) in absolute ethanol (100 mL) under nitrogen was added 5-amino-6-nitroquinoline (5.00 g, 26.4 mmol). With stirring, the solution was placed under vacuum for 30 seconds and then was opened to a hydrogen gas balloon. After stirring for 2 hours under a hydrogen atmosphere, the reaction mixture was evacuated under vacuum and placed under nitrogen, then sonicated for 10 minutes. Methanol was then added to the reaction mixture and the resulting solution was placed under hydrogen atmosphere and stirred for an additional 2 hours. The reaction mixture was then filtered through a Celite pad and the pad washed with methanol (2 × 200 mL). The filtrate was concentrated *in vacuo* and the resulting residue was dissolved in CH₂Cl₂ (75 mL) and the solution was purified via chromatography using an ISCO 330-g Redi-Sep column using 0-10% methanol/CH₂Cl₂ as eluent to provide Compound **3A** as a yellow solid (3.76 g, 89%).

### Step B: Preparation of Compound 3B

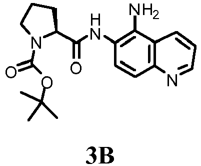

To a solution of 5,6-diaminoquinoline (1.00 g, 6.28 mmol), HATU (2.63 g, 6.91 mmol), *N*,*N*-diisopropylethylamine (3.28 mL, 18.8 mmol) and anhydrous DMF (20 mL) was added Boc-Pro-OH (1.49 g, 6.91 mmol). The reaction was placed under nitrogen atmosphere and allowed to stir at room temperature for 17 hours. The reaction mixture was then partitioned between EtOAc (100 mL) and saturated aqueous NaCl solution (100 mL) and the aqueous layer was extracted with EtOAc (4 × 100 mL). The combined organic extracts were washed with brine (4 × 100 mL), dried over Na₂SO₄, filtered and concentrated *in vacuo*. The residue obtained was dissolved in CH₂Cl₂ (10 mL) and purified via chromatography on an ISCO 80-g Redi-Sep column using 0-5% methanol/CH₂Cl₂ as eluent to provide Compound **3B** as an orange oil (0.713 g, 32%). ESI-LRMS: (M+H-C₄H₉O₂)⁺ = 257.

### Step C - Preparation of Compound 3C

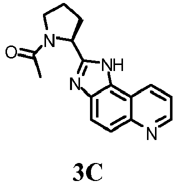

A solution of compound **3B** (3.00 g, 8.41 mmol) in CH₃COOH (70 mL) was put under nitrogen atmosphere, then heated to reflux and allowed to stir at this temperature for 18 hours. The reaction was cooled to room temperature, then concentrated *in vacuo* to provide a brown oil, which was diluted with CH₂Cl₂ and neutralized by adding saturated NaHCO₃ solution (125 mL). The resulting biphasic mixture was stirred for 1 hour, then was diluted with water and extracted with CH₂Cl₂ (2 × 200 mL). The combined organics were dried over MgSO₄, filtered and concentrated *in vacuo* to provide Compound **3C** as an orange foam (2.04 g, 86%). ¹H NMR (CDCl₃) δ 11.61 (br s, 0.32H), 11.04 (br s, 0.68H), 8.93-8.85 (m, 1.68 H), 8.38-8.30 (m, 0.32H), 8.08-7.70 (m, 2H), 7.53-7.40 (m, 1H), 5.51-5.43 (m, 1H), 3.64-3.51 (m, 2H), 3.34-3.13 (m, 1H), 2.51-2.11 (m, 6H). LCMS: (M+H)⁺ = 281.

### Step D - Preparation of Compound 3D

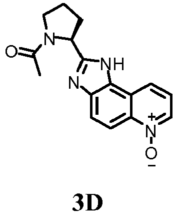

A solution of compound **3C** (2.03 g, 7.24 mmol) in CH₂Cl₂ (75 mL) was placed under nitrogen atmosphere and cooled to 0 °C. To the cooled solution was added 3-chloroperoxybenzoic acid (1.50 g, 8.69 mmol) and the resulting reaction was allowed to warm to room temperature while stirring for 18 hours. The reaction mixture was then cooled to 0 °C and quenched by adding 10% Na₂SO₃ solution (25 mL). The solvent from the reaction mixture was removed *in vacuo* and the remaining aqueous solution was loaded directly onto an ISCO 80 g Redi-Sep column and purified using flash chromatography using 0-10% CH₃OH/CH₂Cl₂ as the eluent to provide a bright yellow foam. The bright yellow foam was then purified again using a second flash chromatography purification using an ISCO 80 g Redi-Sep column and 0-10% CH₃OH/CH₂Cl₂ as the eluent to provide Compound **3D** as a light yellow foam (1.85 g, 86%). ¹H NMR (CDCl₃) δ 11.69 (br s, 0.17H), 11.12 (br s, 0.83H), 8.59-8.38 (m, 2.83H), 8.04-7.96 (d, *J* = 9.5 Hz, 0.17H), 7.88-7.81 (d, *J* = 8.2 Hz, 0.17H), 7.75-7.67 (d, *J* = 9.4 Hz, 0.83H), 7.36-7.23 (m, 1H), 5.43-5.34 (m, 1H), 3.56-3.48 (m, 2H), 3.24-3.06 (m, 1H), 2.43-2.06 (m, 6H).

### Step E - Preparation of Compound 3E

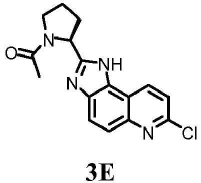

A solution of compound **3D** (1.84 g, 6.20 mmol) in CH₂Cl₂ (20 mL) was placed under nitrogen atmosphere and cooled to 0 °C. To the cooled solution was added triethylamine (1.04 mL, 7.45 mmol), the resulting reaction was stirred for 10 minutes at 0 °C, then a solution of phosphoryl chloride (1.14 g, 7.45 mmol) in CH₂Cl₂ (10 mL) was added dropwise over 10 minutes. The reaction was stirred for an additional 2 hours at 0 °C, then quenched by the dropwise addition of water (3.0 mL). The resulting reaction mixture was neutralized to pH 7 using 2N NaOH (∼ 15 mL), then loaded directly onto a 120 g Redi-Sep column and purified using flash chromatography using 0-10% CH₃OH/CH₂Cl₂ as the eluent to provide a yellow solid product, consisting of two isomers. The two isomers were further separated using semi-preparative HPLC (Luna C18, CH₃CN/water with 0.05% TFA) and the isomerically pure fractions were combined with saturated NaHCO₃ solution (10 mL) and the organic portion was saved. The remaining aqueous portion was extracted with EtOAc (3 × 100 mL) and the combined organics were dried over Na₂SO₄, filtered and concentrated *in vacuo*. The resulting residue was dissolved in a mixture of CH₃CN and water and the solution was freeze-dried overnight to provide Compound **3E** as an off-white solid (463 mg, 23%). ¹H NMR (CDCl₃) δ 11.10 (br s, 1H), 8.87 (br s, 1H), 7.89-7.68 (m, 2H), 7.53-7.42 (d, *J* = 8.6 Hz, 1H), 5.52-5.40 (d, J= 8.0 Hz, 1H), 3.69-3.53 (m, 2H), 3.26 (br s, 1H), 2.52-2.11 (m, 6H).

### EXAMPLE 4

### Preparation of Compound 1

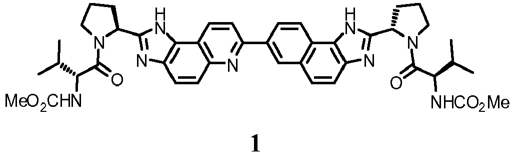

### Step A - Preparation of Compound 4A

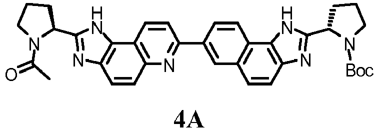

A mixture of Compound **3E** (100 mg, 0.317 mmol), Compound **2A** (150 mg, 0.323 mmol), Pd(PPh₃)₄ (37 mg, 0.032 mmol) and Na₂CO₃ (68 mg, 0.635 mmol) in 2:1 DME:H₂O (6 mL) was heated to 100 °C and allowed to stir at this temperature for 4 hours. The reaction mixture was cooled to room temperature, then diluted with saturated NH₄Cl (50 mL) and extracted with CH₂Cl₂ (2 × 50 mL). The combined organics were concentrated *in vacuo* and the resulting residue was purified using flash chromatography on an ISCO 12 g Redi-Sep column using 0-8% CH₃OH/CH₂Cl₂ as the eluent to provide Compound **4A** as a yellow foam (161 mg, 82%). ¹H NMR (DMSO-*d*₆) δ 13.29-12.52 (m, 2 NH), 8.97-8.83 (m, 2H), 8.63-8.49 (m, 2H), 8.49-8.33 (m, 1H), 8.04-7.68 (m, 4H), 5.43-5.21 (m, 1H), 5.14-4.94 (m, 1H), 3.90-3.42 (m, 4H), 2.43-1.80 (m, 11H), 1.53-0.95 (m, 9H). MS (ESI): (M+H)⁺ = 616.

### Step B - Preparation of Compound 4B

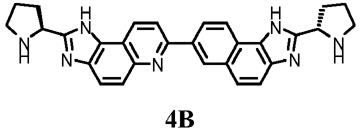

Compound **4A** (159 mg, 0.258 mmol) was dissolved in 6N HCl (8 mL) and the resulting solution was heated to 90 °C and allowed to stir at this temperature for 4 hours. The reaction mixure was then cooled to rrom temperature, concentrated *in vacuo*, and the resulting yellow oily residue was dissolved in a mixture of water (15 mL) and CH₃CN (10 mL). The resulting solution was freeze-dried overnight to provide Compound **4B** as a brownish yellow solid (216 mg, quant.). ¹H NMR (DMSO-*d*₆) δ 10.17 (br s, 2NH), 9.38 (br s, 2NH), 9.06-8.93 (m, 2H), 8.68-8.57 (m, 2H), 8.54-8.45 (m, 1H), 8.15-7.79 (m, 4H), 5.16-4.98 (m, 2H), 2.43-2.00 (m, 12H). MS (ESI): (M+H)⁺ = 474.

### Step C - Reparation of Compound 1

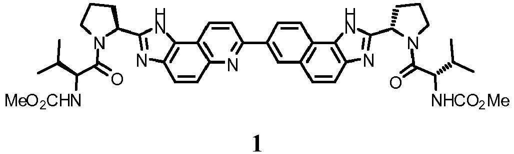

A solution of compound **4B** (157 mg, 0.253 mmol) in DMF (5 mL) was placed under nitrogen atmosphere and cooled to 0 °C. To the cooled solution was added *N*,*N-*diisopropylethylamine (0.39 mL, 2.28 mmol) and the resulting mixture was allowed to stir at 0 °C for 5 minutes. (S)-2-(methoxycarbonylamino)-3-methylbutanoic acid (107 mg, 0.608 mmol) and HATU (232 mg, 0.608 mmol) were then added to the reaction mixture and the resulting reaction was allowed to stir at 0 °C for 1 hour. The reaction mixture was then poured into a mixture of cold water (25 mL), saturated NaHCO₃ solution (25 mL) and EtOAc (50 mL). The resulting suspension was further diluted with brine (50 mL) and EtOAc (25 mL). The layers were partitioned and the aqueous portion extracted with EtOAc (75 mL). The combined organic extracts were concentrated *in vacuo* and the resulting solution of product in remaining DMF was diluted with 100 mL water, heated to 40 °C and allowed to stir overnight while cooling to room temperature on its own.

The resulting suspension was then filtered and the collected brown solid. was purified using flash chromatography on an ISCO 40 g Redi-Sep column using a gradient from 100 % CH₂Cl₂ to 1%NH₄OH/9% CH₃OH/90%CH₂Cl₂ as the eluent. The resulting yellow oil was dissolved in a mixture of CH₃CN and water and the solution was freeze-dried overnight to provide Compound 1 as a yellow solid (99 mg, 51%). ¹H NMR (DMSO-*d*₆) δ 13.3-12.3 (m, 2NH), 8.96-8.74 (m, 2H), 8.64-8.31 (m, 3H), 8.03-7.63 (m, 4H), 7.53-7.17 (m, 2NH), 5.41-5.19 (m, 2H), 4.25-4.04 (m, 2H), 4.03-3.66 (m, 4H), 3.66-3.48 (m, 6H), 2.43-1.77 (m, 10H), 1.09-0.72 (m, 12H). MS (ESI): (M+H)⁺ = 788.8.

### EXAMPLE 5

### Cell-Based HCV Replicon Assay

To measure cell-based anti-HCV activity of selected compounds of the present invention, replicon cells were seeded at 5000 cells/well in 96-well collagen I-coated Nunc plates in the presence of the test compound. Various concentrations of test compound, typically in 10 serial 2-fold dilutions, were added to the assay mixture, with the starting concentration ranging from 250 µM to 1 µM. The final concentration of DMSO was 0.5%, fetal bovine serum was 5%, in the assay media. Cells were harvested on day 3 by the addition of 1x cell lysis buffer (Ambion cat #8721). The replicon RNA level was measured using real time PCR (Taqman assay). The amplicon was located in 5B. The PCR primers were: 5B.2F, ATGGACAGGCGCCCTGA; 5B.2R, TTGATGGGCAGCTTGGTTTC; the probe sequence was FAM-labeled CACGCCATGCGCTGCGG. GAPDH RNA was used as endogenous control and was amplified in the same reaction as NS5B (multiplex PCR) using primers and VIC-labeled probe recommended by the manufacturer (PE Applied Biosystem). The real-time RT-PCR reactions were run on ABI PRISM 7900HT Sequence Detection System using the following program: 48°C and allowed to stir at this temperature for 30 min, 95°C and allowed to stir at this temperature for 10 min, 40 cycles of 95°C and allowed to stir at this temperature for 15 sec, 60°C and allowed to stir at this temperature for 1 minute. The ΔCT values (CT_{5B}-CT_{GAPDH}) were plotted against the concentration of test compound and fitted to the sigmoid dose-response model using XLfit4 (MDL). EC₅₀ was defined as the concentration of inhibitor necessary to achieve ΔCT=1 over the projected baseline; EC₉₀ the concentration necessary to achieve ΔCT=3.2 over the baseline. Alternatively, to quantitate the absolute amount of replicon RNA, a standard curve was established by including serially diluted T7 transcripts of replicon RNA in the Taqman assay. All Taqman reagents were from PE Applied Biosystems. Such an assay procedure was described in detail in *e.g.* Malcolm et al., Antimicrobial Agents and Chemotherapy 50: 1013-1020 (2006).

Using this method, Compound 1 demonstrated an EC₅₀ value of less than 1 nM.

### Uses of the Fused Tricyclic Compounds

The Fused Tricyclic Compounds are useful in human and veterinary medicine for treating or preventing a viral infection or a virus-related disorder in a patient. In accordance with the invention, the Fused Tricyclic Compounds can be administered to a patient in need of treatment or prevention of a viral infection or a virus-related disorder.

Accordingly, in one embodiment, the invention provides use of at least one Fused Tricyclic Compound or a pharmaceutically acceptable salt or solvate thereof for treating a viral infection in a patient. In another embodiment, the invention provides use of at least one Fused Tricyclic Compound or a pharmaceutically acceptable salt or solvate thereof for treating a virus-related disorder in a patient comprising administering to the patient.

### Treatment or Prevention of a Viral Infection

The Fused Tricyclic Compounds can be useful for treating or preventing a viral infection. In one embodiment, the Fused Tricyclic Compounds can be inhibitors of viral replication. In a specific embodiment, the Fused Tricyclic Compounds can be inhibitors of HCV replication. Accordingly, the Fused Tricyclic Compounds are useful for treating viral infections, such as HCV.

Examples of viral infections that can be treated or prevented using the present methods, include but are not limited to, hepatitis A infection, hepatitis B infection and hepatitis C infection.

In one embodiment, the viral infection is hepatitis C infection.

In one embodiment, the hepatitis C infection is acute hepatitis C. In another embodiment, the hepatitis C infection is chronic hepatitis C.

The compositions and combinations of the present invention can be useful for treating a patient suffering from infection related to any HCV genotype. HCV types and subtypes may differ in their antigenicity, level of viremia, severity of disease produced, and response to interferon therapy as described in Holland et al., Pathology, 30(2): 192-195 (1998). The nomenclature set forth in Simmonds et al., J Gen Virol, 74(Pt11):2391-2399 (1993) is widely used and classifies isolates into six major genotypes, 1 through 6, with two or more related subtypes, *e.g*., 1a and 1b. Additional genotypes 7-10 and 11 have been proposed, however the phylogenetic basis on which this classification is based has been questioned, and thus types 7, 8, 9 and 11 isolates have been reassigned as type 6, and type 10 isolates as type 3 (see Lamballerie et al., J Gen Virol, 78(Pt1):45-51 (1997)). The major genotypes have been defined as having sequence similarities of between 55 and 72% (mean 64.5%), and subtypes within types as having 75%-86% similarity (mean 80%) when sequenced in the NS-5 region (see Simmonds et al., J Gen Virol, 75(Pt 5):1053-1061 (1994)).

### Treatment or Prevention of a Virus-Related Disorder

The Fused Tricyclic Compounds can be useful for treating or preventing a virus-related disorder. Accordingly, the Fused Tricyclic Compounds are useful for treating disorders related to the activity of a virus, such as liver inflammation or cirrhosis. Virus-related disorders include, but are not limited to, RNA-dependent polymerase-related disorders and disorders related to HCV infection.

### Treatment or Prevention of a RNA-Dependent Polymerase-Related Disorder

The Fused Tricyclic Compounds can be useful for treating or preventing a RNA dependent polymerase (RdRp) related disorder in a patient. Such disorders include viral infections wherein the infective virus contains a RdRp enzyme.

### Treatment or Prevention of a Disorder Related to HCV Infection

The Fused Tricyclic Compounds can be useful for treating or preventing a disorder related to a HCV infection. Examples of such disorders include, but are not limited to, cirrhosis, portal hypertension, ascites, bone pain, varices, jaundice, hepatic encephalopathy, thyroiditis, porphyria cutanea tarda, cryoglobulinemia, glomerulonephritis, sicca syndrome, thrombocytopenia, lichen planus and diabetes mellitus.

### Combination Therapy

In another embodiment, the present methods for treating or preventing a viral infection or a virus-related disorder can further comprise the administration of one or more additional therapeutic agents which are not Substituted Fused Tricyclic Compounds.

In one embodiment, the additional therapeutic agent is an antiviral agent.

In another embodiment, the additional therapeutic agent is an immunomodulatory agent, such as an immunosuppressive agent.

Accordingly, in one embodiment, the present invention provides treatment of a viral infection in a patient comprising administering to the patient: (i) at least one Substituted Fused Tricyclic Compound, or a pharmaceutically acceptable salt or solvate thereof, and (ii) at least one additional therapeutic agent that is other than a Substituted Fused Tricyclic Compound, wherein the amounts administered are together effective to treat or prevent a viral infection.

When administering a combination therapy of the invention to a patient, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for non-limiting illustration purposes, a Substituted Fused Tricyclic Compound and an additional therapeutic agent may be present in fixed amounts (dosage amounts) in a single dosage unit (*e.g.*, a capsule, a tablet and the like). A commercial example of such single dosage unit containing fixed amounts of two different active compounds is VYTORIN^{®} (available from Merck Schering-Plough Pharmaceuticals, Kenilworth, New Jersey).

In one embodiment, the at least one Substituted Fused Tricyclic Compound is administered during a time when the additional therapeutic agent(s) exert their prophylactic or therapeutic effect, or *vice versa*.

In another embodiment, the at least one Substituted Fused Tricyclic Compound and the additional therapeutic agent(s) are administered in doses commonly employed when such agents are used as monotherapy for treating a viral infection.

In another embodiment, the at least one Substituted Fused Tricyclic Compound and the additional therapeutic agent(s) are administered in doses lower than the doses commonly employed when such agents are used as monotherapy for treating a viral infection.

In still another embodiment, the at least one Substituted Fused Tricyclic Compound and the additional therapeutic agent(s) act synergistically and are administered in doses lower than the doses commonly employed when such agents are used as monotherapy for treating a viral infection.

In one embodiment, the at least one Substituted Fused Tricyclic Compound and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

Viral infections and virus-related disorders that can be treated or prevented using the combination therapy methods of the present invention include, but are not limited to, those listed above.

In one embodiment, the viral infection is HCV infection.

The at least one Substituted Fused Tricyclic Compound and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of at least one Substituted Fused Tricyclic Compound and the additional therapeutic agent(s) may inhibit the resistance of a viral infection to these agents.

Non-limiting examples of additional therapeutic agents useful in the present compositions and treatments include an interferon, an immunomodulator, a viral replication inhibitor, an antisense agent, a therapeutic vaccine, a viral polymerase inhibitor, a nucleoside inhibitor, a viral protease inhibitor, a viral helicase inhibitor, a virion production inhibitor, a viral entry inhibitor, a viral assembly inhibitor, an antibody therapy (monoclonal or polyclonal), and any agent useful for treating an RNA-dependent polymerase-related disorder.

In one embodiment, the additional therapeutic agent is a viral protease inhibitor.

In another embodiment, the additional therapeutic agent is a viral replication inhibitor.

In another embodiment, the additional therapeutic agent is an HCV NS3 protease inhibitor.

In another embodiment, the additional therapeutic agent is an HCV NS5B polymerase inhibitor.

In another embodiment, the additional therapeutic agent is a nucleoside inhibitor.

In another embodiment, the additional therapeutic agent is an interferon.

In one embodiment, the additional therapeutic agent is an HCV replicase inhibitor.

In another embodiment, the additional therapeutic agent is an antisense agent.

In another embodiment, the additional therapeutic agent is a therapeutic vaccine.

In a further embodiment, the additional therapeutic agent is a virion production inhibitor.

In another embodiment, the additional therapeutic agent is an antibody therapy.

In another embodiment, the additional therapeutic agent is an HCV NS2 inhibitor.

In another embodiment, the additional therapeutic agent is an HCV NS4A inhibitor.

In another embodiment, the additional therapeutic agent is an HCV NS4B inhibitor.

In another embodiment, the additional therapeutic agent is an HCV NS5A inhibitor

In another embodiment, the additional therapeutic agent is an HCV NS3 helicase inhibitor.

In another embodiment, the additional therapeutic agent is an HCV IRES inhibitor.

In another embodiment, the additional therapeutic agent is an HCV p7 inhibitor.

In another embodiment, the additional therapeutic agent is an HCV entry inhibitor.

In another embodiment, the additional therapeutic agent is an HCV assembly inhibitor.

In one embodiment, the additional therapeutic agents comprise a protease inhibitor and a polymerase inhibitor.

In still another embodiment, the additional therapeutic agents comprise a protease inhibitor and an immunomodulatory agent.

In yet another embodiment, the additional therapeutic agents comprise a polymerase inhibitor and an immunomodulatory agent.

In another embodiment, the additional therapeutic agents comprise a protease inhibitor and a nucleoside.

In another embodiment, the additional therapeutic agents comprise an immunomodulatory agent and a nucleoside.

In one embodiment, the additional therapeutic agents comprise a protease inhibitor and a NS5A inhibitor.

In another embodiment, the additional therapeutic agents comprise a nucleoside and a NS5A inhibitor.

In another embodiment, the additional therapeutic agents comprise a protease inhibitor, an immunomodulatory agent and a nucleoside.

In still another embodiment, the additional therapeutic agents comprise a protease inhibitor, a nucleoside and a NS5A inhibitor.

In a further embodiment, the additional therapeutic agents comprise a protease inhibitor, a polymerase inhibitor and an immunomodulatory agent.

In another embodiment, the additional therapeutic agent is ribavirin.

HCV polymerase inhibitors useful in the present compositions and treatments include, but are not limited to, VP-19744 (Wyeth/ViroPharma), PSI-7851 (Pharmasset), R7128 (Roche/Pharmasset), PF-868554/filibuvir (Pfizer), VCH-759 (ViroChem Pharma), HCV-796 (Wyeth/ViroPharma), IDX-184 (Idenix), IDX-375 (Idenix), NM-283 (Idenix/Novartis), R-1626 (Roche), MK-0608 (Isis/Merck), INX-8014 (Inhibitex), INX-8018 (Inhibitex), INX-189 (Inhibitex), GS 9190 (Gilead), A-848837 (Abbott), ABT-333 (Abbott), ABT-072 (Abbott), A-837093 (Abbott), BI-207127 (Boehringer-Ingelheim), BILB-1941 (Boehringer-Ingelheim), MK-3281 (Merck), VCH222 (ViroChem), VCH916 (ViroChem), VCH716(ViroChem), GSK-71185 (Glaxo SmithKline), ANA598 (Anadys), GSK-625433 (Glaxo SmithKline), XTL-2125 (XTL Biopharmaceuticals), and those disclosed in Ni et al., Current Opinion in Drug Discovery and Development, 7(4):446 (2004); Tan et al., Nature Reviews, 1:867 (2002); and Beaulieu et al., Current Opinion in Investigational Drugs, 5:838 (2004).

Other HCV polymerase inhibitors useful in the present compositions and treatments include, but are not limited to, those disclosed in International Publication Nos. WO 08/082484, WO 08/082488, WO 08/083351, WO 08/136815, WO 09/032116, WO 09/032123, WO 09/032124 and WO 09/032125.

Interferons useful in the present compositions and treatments include, but are not limited to, interferon alfa-2a, interferon alfa-2b, interferon alfacon-1 and PEG-interferon alpha conjugates. "PEG-interferon alpha conjugates" are interferon alpha molecules covalently attached to a PEG molecule. Illustrative PEG-interferon alpha conjugates include interferon alpha-2a (Roferon^{™}, Hoffman La-Roche, Nutley, New Jersey) in the form of pegylated interferon alpha-2a (*e.g.*, as sold under the trade name Pegasys^{™}), interferon alpha-2b (Intron^{™}, from Schering-Plough Corporation) in the form of pegylated interferon alpha-2b (*e.g.*, as sold under the trade name PEG-Intron^{™} from Schering-Plough Corporation), interferon alpha-2b-XL (*e.g.*, as sold under the trade name PEG-Intron^{™}), interferon alpha-2c (Berofor Alpha^{™}, Boehringer Ingelheim, Ingelheim, Germany), PEG-interferon lambda (Bristol-Myers Squibb and ZymoGenetics), interferon alfa-2b alpha fusion polypeptides, interferon fused with the human blood protein albumin (Albuferon^{™}, Human Genome Sciences), Omega Interferon (Intarcia), Locteron controlled release interferon (Biolex/OctoPlus), , Biomed-510 (omega interferon), Peg-IL-29 (ZymoGenetics), Locteron CR (Octoplus), IFN-α-2b-XL (Flamel Technologies), and consensus interferon as defined by determination of a consensus sequence of naturally occurring interferon alphas (Infergen^{™}, Amgen, Thousand Oaks, California).

Antibody therapy agents useful in the present compositions and treatments include, but are not limited to, antibodies specific to IL-10 (such as those disclosed in US Patent Publication No. US2005/0101770, humanized 12G8, a humanized monoclonal antibody against human IL-10, plasmids containing the nucleic acids encoding the humanized 12G8 light and heavy chains were deposited with the American Type Culture Collection (ATCC) as deposit numbers PTA-5923 and PTA-5922, respectively), and the like).

Examples of viral protease inhbitors useful in the present compositions and treatments include, but are not limited to, an HCV protease inhibitor.

HCV protease inhibitors useful in the present compositions and treatments include, but are not limited to, those disclosed in U.S. Patent Nos. 7,494,988, 7,485,625, 7,449,447, 7,442,695, 7,425,576, 7,342,041, 7,253,160, 7,244,721, 7,205,330, 7,192,957, 7,186,747, 7,173,057, 7,169,760, 7,012,066, 6,914,122, 6,911,428, 6,894,072, 6,846,802, 6,838,475, 6,800,434, 6,767,991, 5,017,380, 4,933,443, 4,812,561 and 4,634,697; U.S. Patent Publication Nos. US20020068702, US20020160962, US20050119168, US20050176648, US20050209164, US20050249702 and US20070042968; and International Publication Nos. WO 03/006490, WO 03/087092, WO 04/092161 and WO 08/124148.

Additional HCV protease inhibitors useful in the present compositions and treatments include, but are not limited to, SCH503034 (Boceprevir, Schering-Plough), SCH900518 (Schering-Plough), VX-950 (Telaprevir, Vertex), VX-500 (Vertex), VX-813 (Vertex), VBY-376 (Virobay), BI-201335 (Boehringer Ingelheim), TMC-435 (Medivir/Tibotec), ABT-450 (Abbott), MK-7009 (Merck), TMC-435350 (Medivir), ITMN-191/R7227 (InterMune/Roche), EA-058 (Abbott/Enanta), EA-063 (Abbott/Enanta), GS-9132 (Gilead/Achillion), ACH-1095 (Gilead/Achillon), IDX-136 (Idenix), IDX-316 (Idenix), ITMN-8356 (InterMune), ITMN-8347 (InterMune), ITMN-8096 (InterMune), ITMN-7587 (InterMune), PHX1766 (Phenomix), amprenavir, atazanavir, fosemprenavir, indinavir, lopinavir, ritonavir, nelfinavir, saquinavir, tipranavir, Kaletra (a combination of ritonavir and lopinavir) and TMC114.

Additional examples of HCV protease inhbitors useful in the present compositions and treatments include, but are not limited to, those disclosed in Landro et al., Biochemistry, 36(31):9340-9348 (1997); Ingallinella et al., Biochemistry, 37(25):8906-8914 (1998); Llinas-Brunet et al., Bioorg Med Chem Lett, 8(13):1713-1718 (1998); Martin et al., Biochemistry, 37(33):111459-11468 (1998); Dimasi et al., J Virol, 71(10):7461-7469 (1997); Martin et al., Protein Eng, 10(5):607-614 (1997); Elzouki et al., J Hepat, 27(1):42-48 (1997); BioWorld Today, 9(217):4 (November 10, 1998); U.S. Patent Publication Nos. US2005/0249702 and US 2007/0274951; and International Publication Nos. WO 98/14181, WO 98/17679, WO 98/17679, WO 98/22496 and WO 99/07734 and WO 05/087731.

Further examples of HCV protease inhibitors useful in the present compositions and treatments include, but are not limited to, the following compounds:

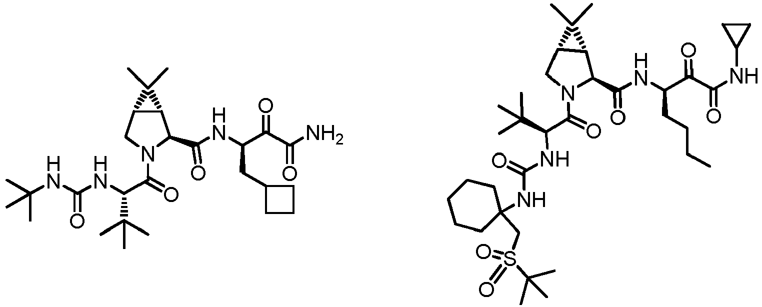

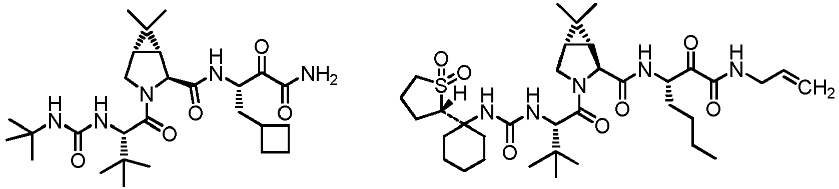

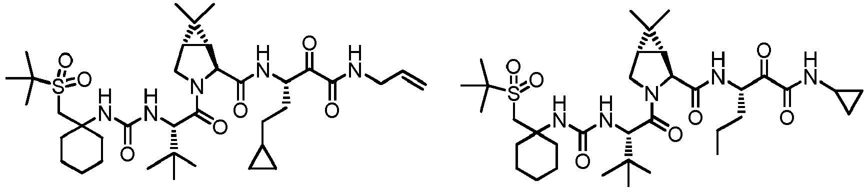

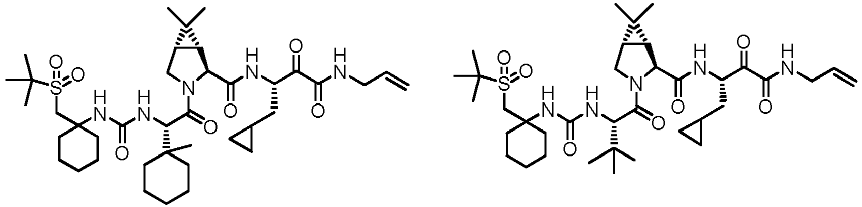

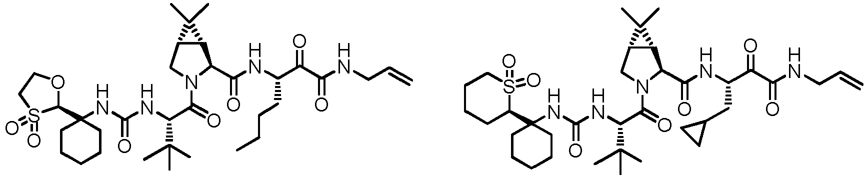

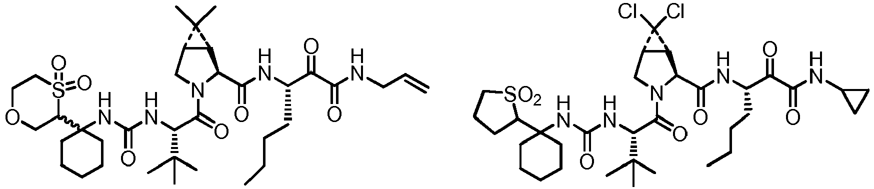

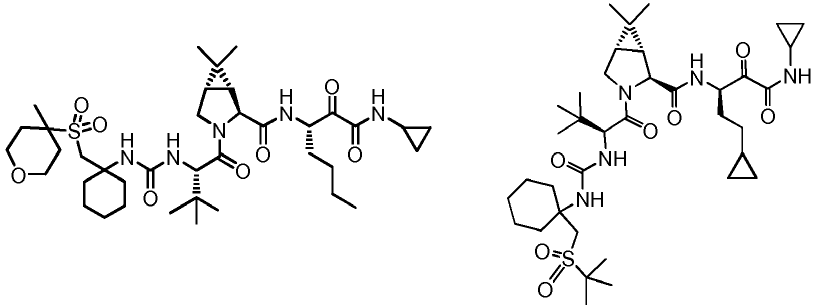

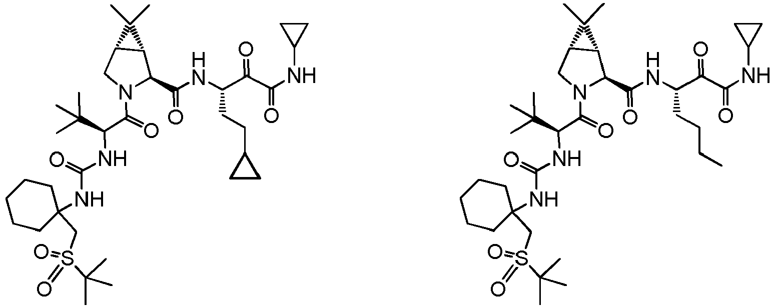

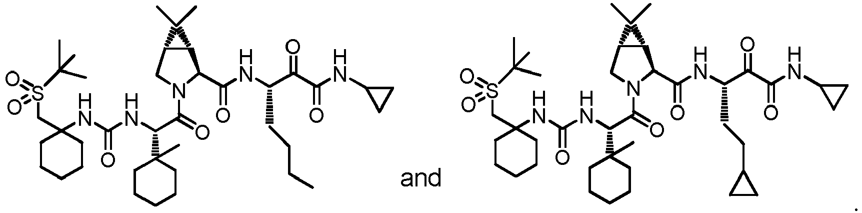

Viral replication inhibitors useful in the present compositions and treatments include, but are not limited to, HCV replicase inhibitors, IRES inhibitors, NS4A inhibitors, NS3 helicase inhibitors, NS5A inhibitors, ribavirin, AZD-2836 (Astra Zeneca), BMS-790052 (Bristol-Myers Squibb), viramidine, A-831 (Arrow Therapeutics); an antisense agent or a therapeutic vaccine.

In one embodiment, viral replication inhibitors useful in the present compositions and treatments include, but are not limited to, HCV replicase inhibitors, IRES inhibitors, NS4A inhibitors, NS3 helicase inhibitors and NS5A inhibitors.

HCV NS4A inhibitors useful in the useful in the present compositions and treatments include, but are not limited to, those disclosed in U.S. Patent Nos. 7,476,686 and 7,273,885; U.S. Patent Publication No. US20090022688; and International Publication Nos. WO 2006/019831 and WO 2006/019832. Additional HCV NS4A inhibitors useful in the useful in the present compositions and treatments include, but are not limited to, AZD2836 (Astra Zeneca) and ACH-806 (Achillon Pharmaceuticals, New Haven, CT).

HCV replicase inhibitors useful in the useful in the present compositions and treatments include, but are not limited to, those disclosed in U.S. Patent Publication No. US20090081636.

Therapeutic vaccines useful in the present compositions and treatments include, but are not limited to, IC41 (Intercell Novartis), CSL123 (Chiron/CSL), GI 5005 (Globeimmune), TG-4040 (Transgene), GNI-103 (GENimmune), Hepavaxx C (ViRex Medical), ChronVac-C (Inovio/Tripep), PeviPROTM (Pevion Biotect), HCV/MF59 (Chiron/Novartis) and Civacir (NABI).

Examples of further additional therapeutic agents useful in the present compositions and treatments include, but are not limited to, TT033 (Benitec/Tacere Bio/Pfizer), Sirna-034 (Sirna Therapeutics), GNI-104 (GENimmune), GI-5005 (GlobeImmune), IDX-102 (Idenix), Levovirin^{™} (ICN Pharmaceuticals, Costa Mesa, California); Humax (Genmab), ITX-2155 (Ithrex/Novartis), PRO 206 (Progenics), HepaCide-I (NanoVirocides), MX3235 (Migenix), SCY-635 (Scynexis); KPE02003002 (Kemin Pharma), Lenocta (VioQuest Pharmaceuticals), IET - Interferon Enhancing Therapy (Transition Therapeutics), Zadaxin (SciClone Pharma), VP 50406^{™} (Viropharma, Incorporated, Exton, Pennsylvania); Taribavirin (Valeant Pharmaceuticals); Nitazoxanide (Romark); Debio 025 (Debiopharm); GS-9450 (Gilead); PF-4878691 (Pfizer); ANA773 (Anadys); SCV-07 (SciClone Pharmaceuticals); NIM-881 (Novartis); ISIS 14803^{™} (ISIS Pharmaceuticals, Carlsbad, California); Heptazyme^{™} (Ribozyme Pharmaceuticals, Boulder, Colorado); Thymosin^{™} (SciClone Pharmaceuticals, San Mateo, California); Maxamine^{™} (Maxim Pharmaceuticals, San Diego, California); NKB-122 (JenKen Bioscience Inc., North Carolina); Alinia (Romark Laboratories), INFORM-1 (a combination of R7128 and ITMN-191); and mycophenolate mofetil (Hoffman-LaRoche, Nutley, New Jersey).

The doses and dosage regimen of the other agents used in the combination therapies of the present invention for the treatment or prevention of a viral infection or virus-related disorder can be determined by the attending clinician, taking into consideration the approved doses and dosage regimen in the package insert; the age, sex and general health of the patient; and the type and severity of the viral infection or related disease or disorder. When administered in combination, the Substituted Fused Tricyclic Compound(s) and the other agent(s) can be administered simultaneously (*i.e.*, in the same composition or in separate compositions one right after the other) or sequentially. This is particularly useful when the components of the combination are given on different dosing schedules, *e.g*., one component is administered once daily and another every six hours, or when the preferred pharmaceutical compositions are different, *e.g.*, one is a tablet and one is a capsule. A kit comprising the separate dosage forms is therefore advantageous.

Generally, a total daily dosage of the at least one Substituted Fused Tricyclic Compound(s) alone, or when administered as combination therapy, can range from about 1 to about 2500 mg per day, although variations will necessarily occur depending on the target of therapy, the patient and the route of administration. In one embodiment, the dosage is from about 10 to about 1000 mg/day, administered in a single dose or in 2-4 divided doses. In another embodiment, the dosage is from about 1 to about 500 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In another embodiment, the dosage is from about 500 to about 1500 mg/day, administered in a single dose or in 2-4 divided doses. In still another embodiment, the dosage is from about 500 to about 1000 mg/day, administered in a single dose or in 2-4 divided doses. In yet another embodiment, the dosage is from about 100 to about 500 mg/day, administered in a single dose or in 2-4 divided doses.

In one embodiment, when the additional therapeutic agent is INTRON-A interferon alpha 2b (commercially available from Schering-Plough Corp.), this agent is administered by subcutaneous injection at 3MIU(12 mcg)/0.5mL/TIW for 24 weeks or 48 weeks for first time treatment.

In another embodiment, when the additional therapeutic agent is PEG-INTRON interferon alpha 2b pegylated (commercially available from Schering-Plough Corp.), this agent is administered by subcutaneous injection at 1.5 mcg/kg/week, within a range of 40 to 150 mcg/week, for at least 24 weeks.

In another embodiment, when the additional therapeutic agent is ROFERON A interferon alpha 2a (commercially available from Hoffmann-La Roche), this agent is administered by subcutaneous or intramuscular injection at 3MIU(11.1 mcg/mL)/TIW for at least 48 to 52 weeks, or alternatively 6MIU/TIW for 12 weeks followed by 3MIU/TIW for 36 weeks.

In still another embodiment, when the additional therapeutic agent is PEGASUS interferon alpha 2a pegylated (commercially available from Hoffmann-La Roche), this agent is administered by subcutaneous injection at 180 mcg/1mL or 180 mcg/0.5mL, once a week for at least 24 weeks.

In yet another embodiment, when the additional therapeutic agent is INFERGEN interferon alphacon-1 (commercially available from Amgen), this agent is administered by subcutaneous injection at 9 mcg/TIW is 24 weeks for first time treatment and up to 15 mcg/TIW for 24 weeks for non-responsive or relapse treatment.

In a further embodiment, when the additional therapeutic agent is Ribavirin (commercially available as REBETOL ribavirin from Schering-Plough or COPEGUS ribavirin from Hoffmann-La Roche), this agent is administered at a daily dosage of from about 600 to about 1400 mg/day for at least 24 weeks.

In one embodiment, one or more compounds of the present invention are administered with one or more additional therapeutic agents selected from an HCV protease inhibitor, an HCV replication inhibitor, a nucleoside, an interferon, a pegylated interferon and ribavirin. The combination therapies can include any combination of these additional therapeutic agents.

In another embodiment, one or more compounds of the present invention are administered with one additional therapeutic agent selected from an HCV protease inhibitor, an HCV replication inhibitor, a nucleoside, an interferon, a pegylated interferon and ribavirin.

In another embodiment, one or more compounds of the present invention are administered with two additional therapeutic agents selected from an HCV protease inhibitor, an HCV replication inhibitor, a nucleoside, an interferon, a pegylated interferon and ribavirin.

In a specific embodiment, one or more compounds of the present invention are administered with an HCV protease inhibitor and ribavirin. In another specific embodiment, one or more compounds of the present invention are administered with a pegylated interferon and ribavirin.

In another embodiment, one or more compounds of the present invention are administered with three additional therapeutic agents selected from an HCV protease inhibitor, an HCV replication inhibitor, a nucleoside, an interferon, a pegylated interferon and ribavirin.

In one embodiment, one or more compounds of the present invention are administered with one or more additional therapeutic agents selected from an HCV polymerase inhibitor, a viral protease inhibitor, an interferon, and a viral replication inhibitor. In another embodiment, one or more compounds of the present invention are administered with one or more additional therapeutic agents selected from an HCV polymerase inhibitor, a viral protease inhibitor, an interferon, and a viral replication inhibitor. In another embodiment, one or more compounds of the present invention are administered with one or more additional therapeutic agents selected from an HCV polymerase inhibitor, a viral protease inhibitor, an interferon, and ribavirin.

In one embodiment, one or more compounds of the present invention are administered with one additional therapeutic agent selected from an HCV polymerase inhibitor, a viral protease inhibitor, an interferon, and a viral replication inhibitor. In another embodiment, one or more compounds of the present invention are administered with ribavirin.

In one embodiment, one or more compounds of the present invention are administered with two additional therapeutic agents selected from an HCV polymerase inhibitor, a viral protease inhibitor, an interferon, and a viral replication inhibitor.

In another embodiment, one or more compounds of the present invention are administered with ribavirin, interferon and another therapeutic agent.

In another embodiment, one or more compounds of the present invention are administered with ribavirin, interferon and another therapeutic agent, wherein the additional therapeutic agent is selected from an HCV polymerase inhibitor, a viral protease inhibitor, and a viral replication inhibitor.

In still another embodiment, one or more compounds of the present invention are administered with ribavirin, interferon and a viral protease inhibitor.

In another embodiment, one or more compounds of the present invention are administered with ribavirin, interferon and an HCV protease inhibitor.

In another embodiment, one or more compounds of the present invention are administered with ribavirin, interferon and boceprevir or telaprevir.

In a further embodiment, one or more compounds of the present invention are administered with ribavirin, interferon and an HCV polymerase inhibitor.

### Compositions and Administration

Due to their activity, the Fused Tricyclic Compounds are useful in veterinary and human medicine. As described above, the Fused Tricyclic Compounds are useful for treating or preventing a viral infection or a virus-related disorder in a patient in need thereof

When administered to a patient, the Fused Tricyclic Compounds can be administered as a component of a composition that comprises a pharmaceutically acceptable carrier or vehicle. The present invention provides pharmaceutical compositions comprising an effective amount of at least one Fused Tricyclic Compound and a pharmaceutically acceptable carrier. In the pharmaceutical compositions and methods of the present invention, the active ingredients will typically be administered in admixture with suitable carrier materials suitably selected with respect to the intended form of administration, *i.e.*, oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Solid form preparations include powders, tablets, dispersible granules, capsules, cachets and suppositories. Powders and tablets may be comprised of from about 0.5 to about 95 percent inventive composition. Tablets, powders, cachets and capsules can be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

The Fused Tricyclic Compounds of the present invention may also be deliverable transdermally. The transdermal compositions can take the form of creams, lotions, aerosols and/or emulsions and can be included in a transdermal patch of the matrix or reservoir type as are conventional in the art for this purpose.

Additionally, the compositions of the present invention may be formulated in sustained release form to provide the rate controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Fused Tricyclic Compounds are administered orally.

In another embodiment, the one or more Fused Tricyclic Compounds are administered intravenously.

In another embodiment, the one or more Fused Tricyclic Compounds are administered topically.

In still another embodiment, the one or more Fused Tricyclic Compounds are administered sublingually.

In one embodiment, a pharmaceutical preparation comprising at least one Fused Tricyclic Compound is in unit dosage form. In such form, the preparation is subdivided into unit doses containing effective amounts of the active components.

Compositions can be prepared according to conventional mixing, granulating or coating methods, respectively, and the present compositions can contain, in one embodiment, from about 0.1% to about 99% of the Fused Tricyclic Compound(s) by weight or volume. In various embodiments, the present compositions can contain, in one embodiment, from about 1% to about 70% or from about 5% to about 60% of the Fused Tricyclic Compound(s) by weight or volume.

The quantity of Fused Tricyclic Compound in a unit dose of preparation may be varied or adjusted from about 1 mg to about 2500 mg. In various embodiment, the quantity is from about 10 mg to about 1000 mg, 1 mg to about 500 mg, 1 mg to about 100 mg, and 1 mg to about 100 mg.

For convenience, the total daily dosage may be divided and administered in portions during the day if desired. In one embodiment, the daily dosage is administered in one portion. In another embodiment, the total daily dosage is administered in two divided doses over a 24 hour period. In another embodiment, the total daily dosage is administered in three divided doses over a 24 hour period. In still another embodiment, the total daily dosage is administered in four divided doses over a 24 hour period.

The amount and frequency of administration of the Fused Tricyclic Compounds will be regulated according to the judgment of the attending clinician considering such factors as age, condition and size of the patient as well as severity of the symptoms being treated. Generally, a total daily dosage of the Fused Tricyclic Compounds range from about 0.1 to about 2000 mg per day, although variations will necessarily occur depending on the target of therapy, the patient and the route of administration. In one embodiment, the dosage is from about 1 to about 200 mg/day, administered in a single dose or in 2-4 divided doses. In another embodiment, the dosage is from about 10 to about 2000 mg/day, administered in a single dose or in 2-4 divided doses. In another embodiment, the dosage is from about 100 to about 2000 mg/day, administered in a single dose or in 2-4 divided doses. In still another embodiment, the dosage is from about 500 to about 2000 mg/day, administered in a single dose or in 2-4 divided doses.

The compositions of the invention can further comprise one or more additional therapeutic agents, selected from those listed above herein. Accordingly, in one embodiment, the present invention provides compositions comprising: (i) at least one Fused Tricyclic Compound or a pharmaceutically acceptable salt, solvate, ester or prodrug thereof; (ii) one or more additional therapeutic agents that are not a Fused Tricyclic Compound; and (iii) a pharmaceutically acceptable carrier, wherein the amounts in the composition are together effective to treat a viral infection or a virus-related disorder.

### Kits

In one aspect, the present invention provides a kit comprising a therapeutically effective amount of at least one Fused Tricyclic Compound, or a pharmaceutically acceptable salt, solvate, ester or prodrug of said compound and a pharmaceutically acceptable carrier, vehicle or diluent.

In another aspect the present invention provides a kit comprising an amount of at least one Fused Tricyclic Compound, or a pharmaceutically acceptable salt, solvate, ester or prodrug of said compound and an amount of at least one additional therapeutic agent listed above, wherein the amounts of the two or more active ingredients result in a desired therapeutic effect. In one embodiment, the one or more Fused Tricyclic Compounds and the one or more additional therapeutic agents are provided in the same container. In one embodiment, the one or more Fused Tricyclic Compounds and the one or more additional therapeutic agents are provided in separate containers.

## Claims

1. A compound having the formula (I): 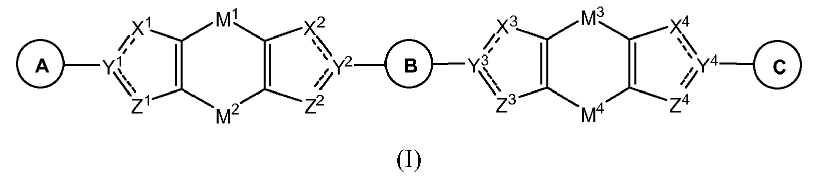 or a pharmaceutically acceptable salt or solvate thereof, wherein the group: 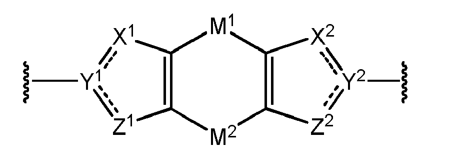 has the structure: 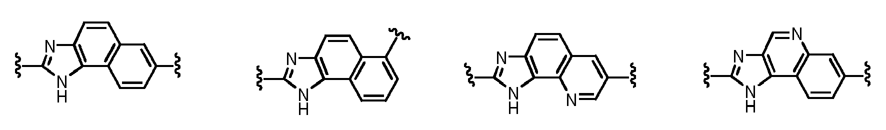 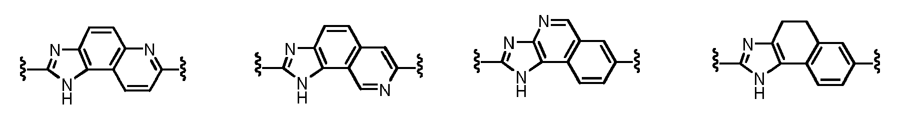 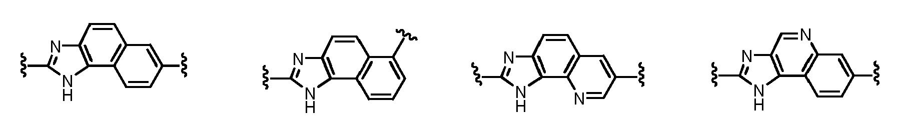 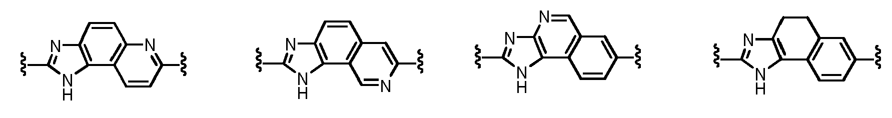 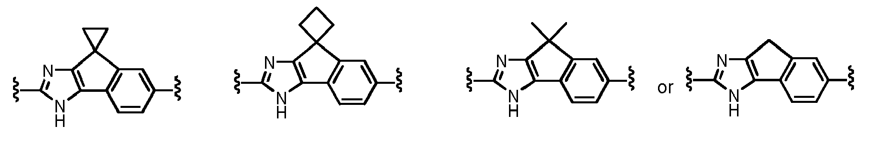 the group: 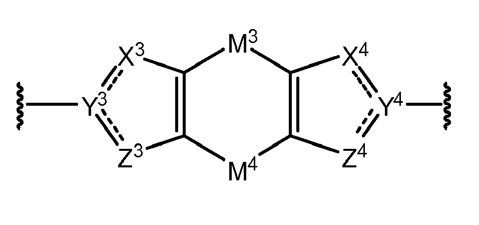 has the structure: 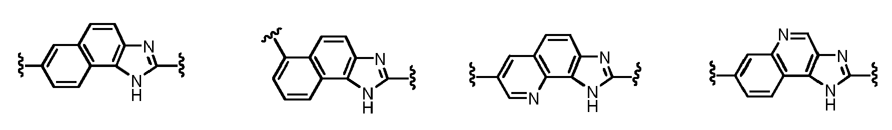 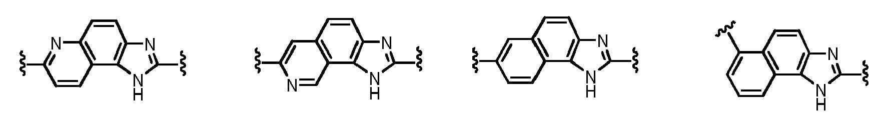 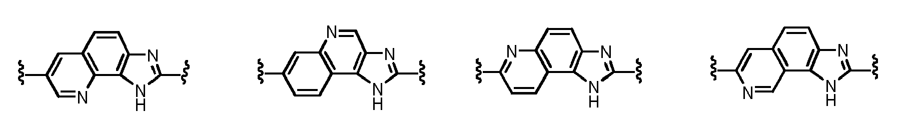 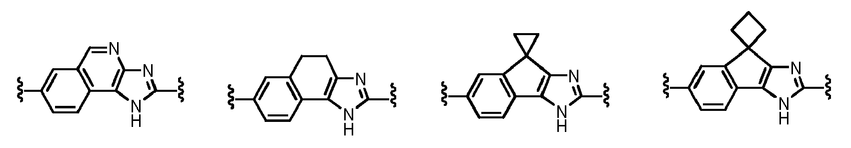 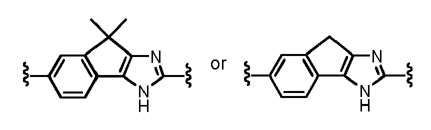 A and C are each: 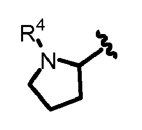 wherein each occurrence of R⁴ is independently: 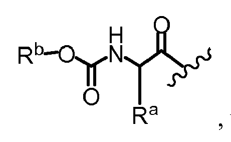 wherein R^{a} is H, alkyl, haloalkyl, cycloalkyl or aryl, and R^{b} is alkyl, haloalkyl or cycloalkyl and
B is a bond or phenylene;
such that at least one of the rings containing: (i) X², Y² and Z² or (ii) X³, Y³ and Z³ is other than a benzene ring.

2. The compound of claim 1, wherein each occurrence of R⁴ is independently selected from:

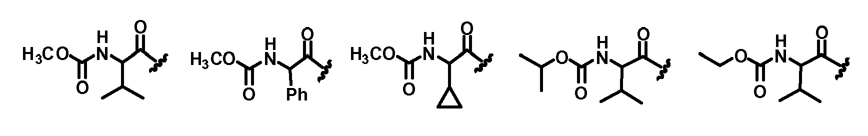

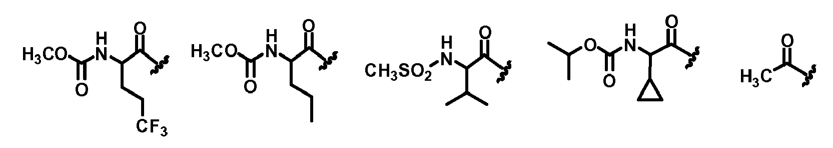

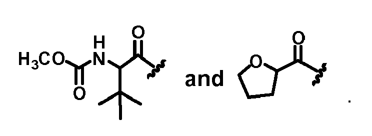

3. The compound of claim 2, wherein each occurrence of R⁴ is:

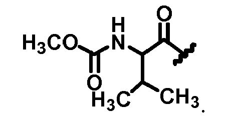

4. The compound of claim 3, wherein B is a bond.

5. A compound according to claim 1 having the structure: 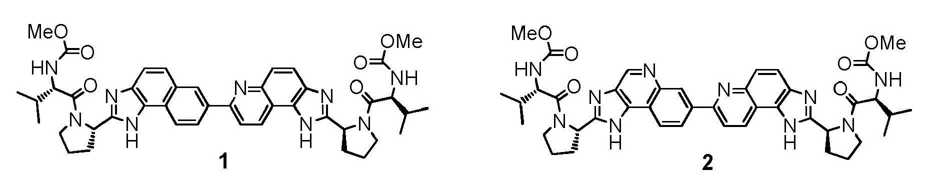 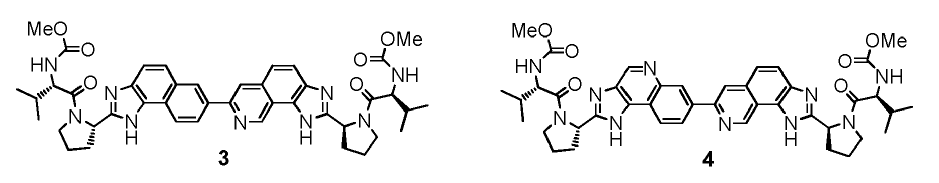 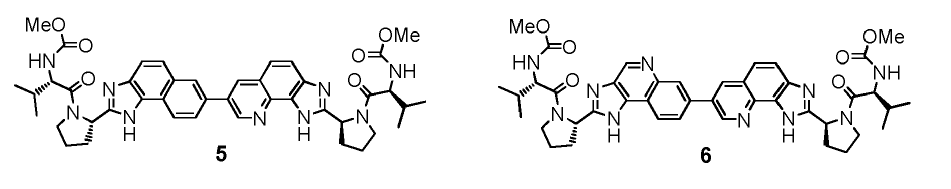 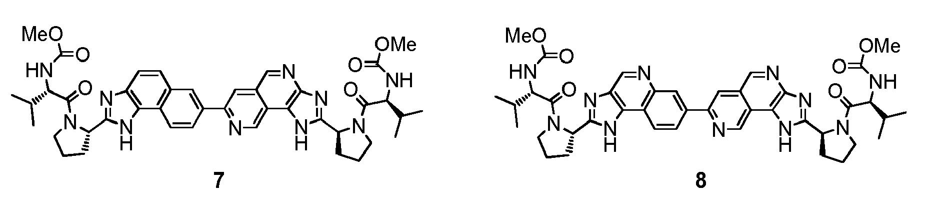 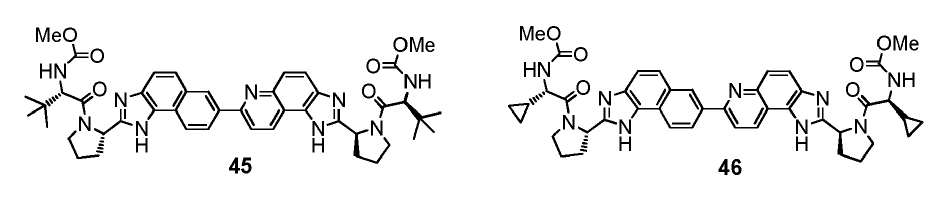 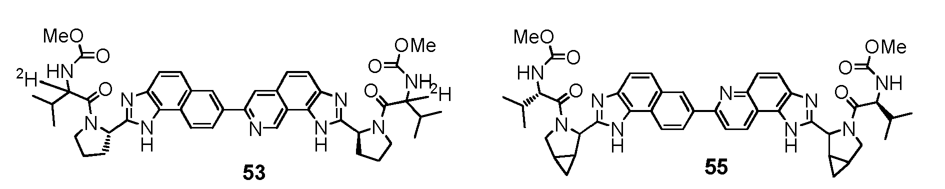 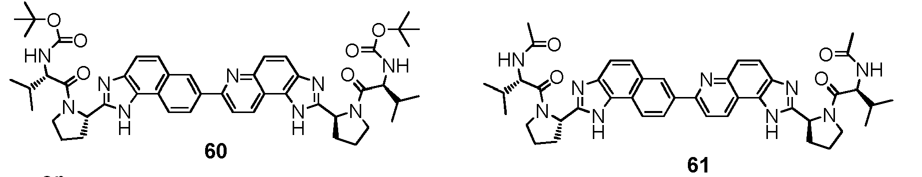 or 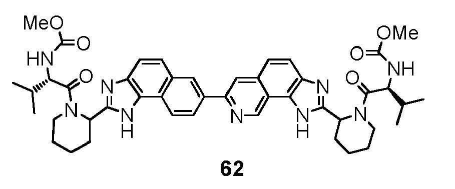 or a pharmaceutically acceptable salt or solvate thereof.

6. A pharmaceutical composition comprising at least one compound of claim 1 or a pharmaceutically acceptable salt or solvate thereof, and at least one pharmaceutically acceptable carrier.

7. The pharmaceutical composition of claim 6, further comprising at least one additional therapeutic agent, wherein the at least one additional therapeutic agent is not a compound of claim 1, wherein the at least one additional therapeutic agent is selected from: an interferon, an immunomodulator, a viral replication inhibitor, an antisense agent, a therapeutic vaccine, a viral polymerase inhibitor, a nucleoside inhibitor, a viral protease inhibitor, a viral helicase inhibitor, a virion production inhibitor, a viral entry inhibitor, a viral assembly inhibitor, an antibody therapy (monoclonal or polyclonal), and any agent useful for treating an RNA-dependent polymerase-related disorder.

8. A compound of claim 1, or a pharmaceutically acceptable salt or solvate thereof, for use in treating a viral infection in a patient.

9. The compound for use of claim 8, further comprising administering to the patient at least one additional therapeutic agent, wherein the at least one additional therapeutic agent is not a compound of claim 1, wherein the at least one additional therapeutic agent is selected from: an interferon, an immunomodulator, a viral replication inhibitor, an antisense agent, a therapeutic vaccine, a viral polymerase inhibitor, a nucleoside inhibitor, a viral protease inhibitor, a viral helicase inhibitor, a virion production inhibitor, a viral entry inhibitor, a viral assembly inhibitor, an antibody therapy (monoclonal or polyclonal), and any agent useful for treating an RNA-dependent polymerase-related disorder.

## Patentansprüche

1. Eine Verbindung der Formel (I): 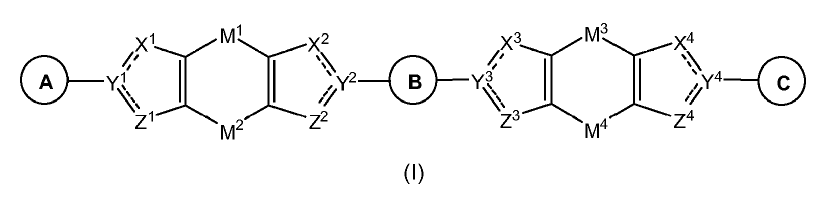 oder ein pharmazeutisch annehmbares Salz oder Solvat davon, wobei die Gruppe: 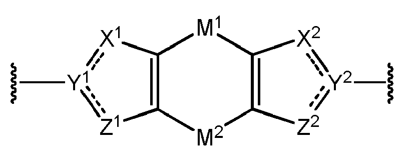 die Struktur: 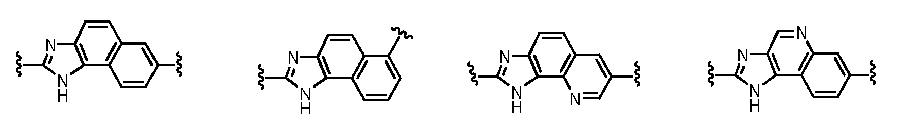 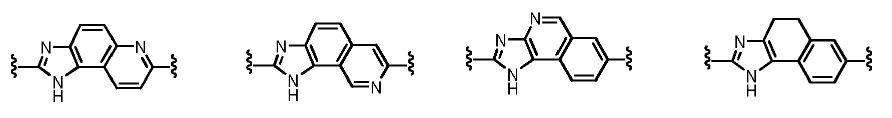 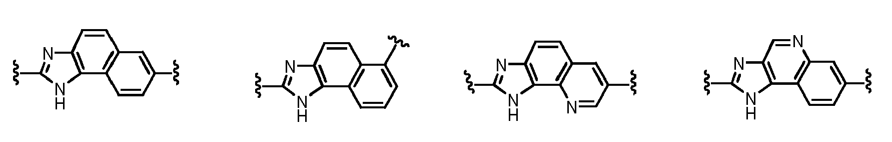 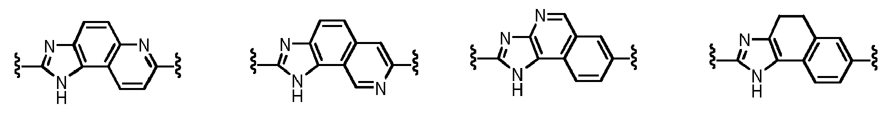 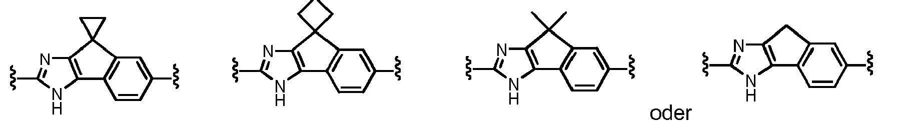 besitzt,
die Gruppe: 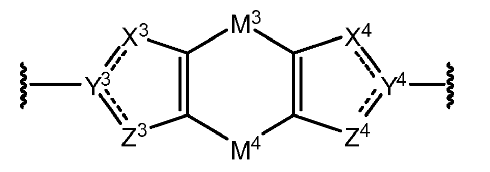 die Struktur: 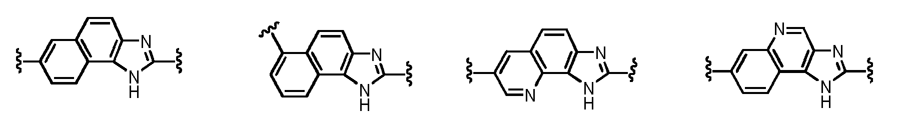 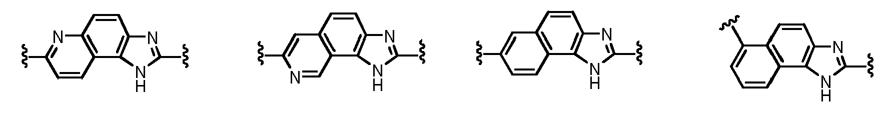 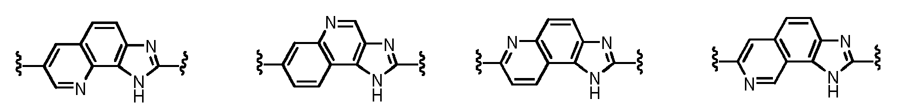 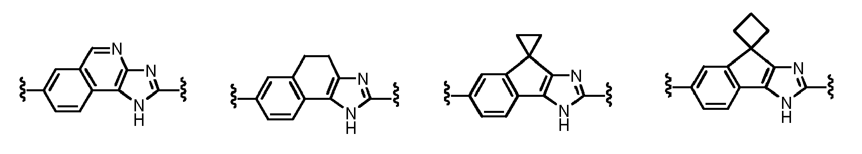 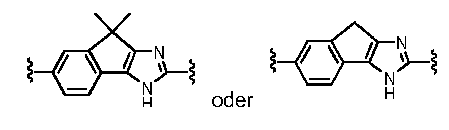 besitzt,
A und C jeweils: 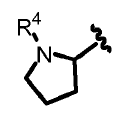 sind,
wobei jedes Vorkommen von R⁴ unabhängig: 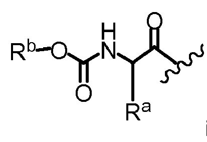 ist, wobei R^{a} H, Alkyl, Halogenalkyl, Cycloalkyl oder Aryl ist und R^{b} Alkyl, Halogenalkyl oder Cycloalkyl ist, und
B eine Bindung oder Phenylen ist,
so dass wenigstens einer der Ringe, enthaltend: (i) X², Y² und Z² oder (ii) X³, Y³ und Z³, anders als ein Benzolring ist.

2. Die Verbindung nach Anspruch 1, wobei jedes Vorkommen von R⁴ unabhängig ausgewählt ist aus:

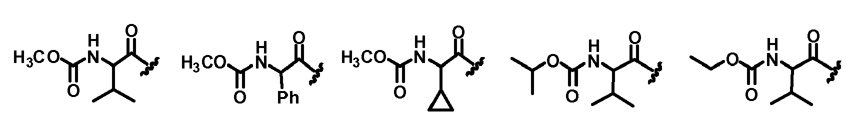

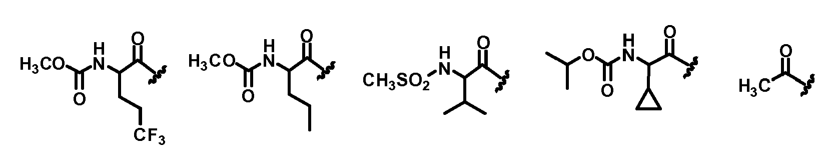

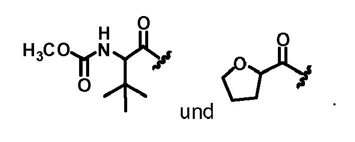

3. Die Verbindung nach Anspruch 2, wobei jedes Vorkommen von R⁴ ist:

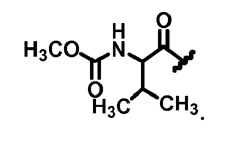

4. Die Verbindung nach Anspruch 3, wobei B eine Bindung ist.

5. Eine Verbindung gemäß Anspruch 1 mit der Struktur: 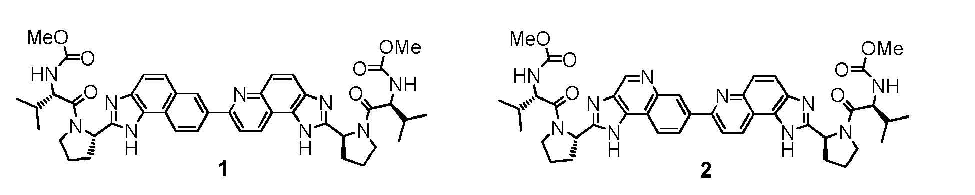 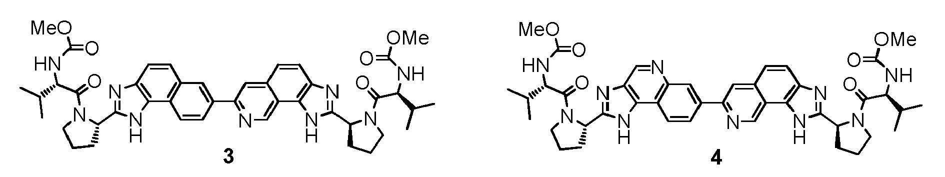 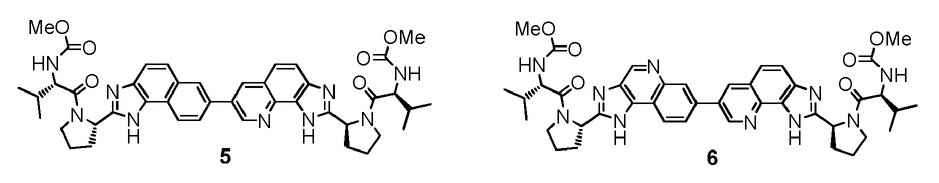 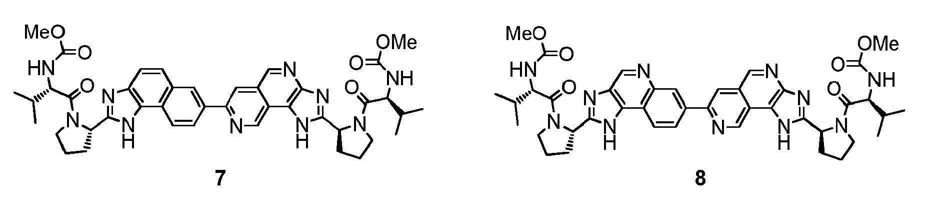 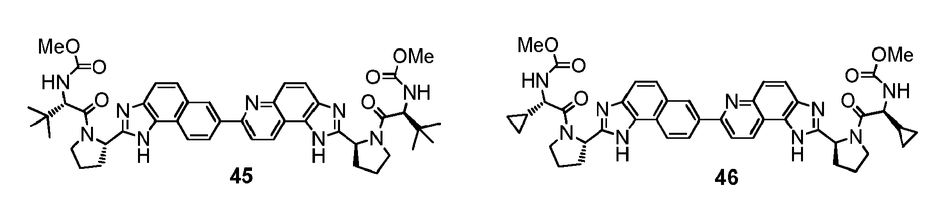 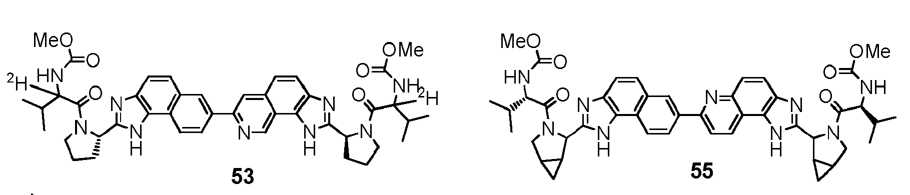 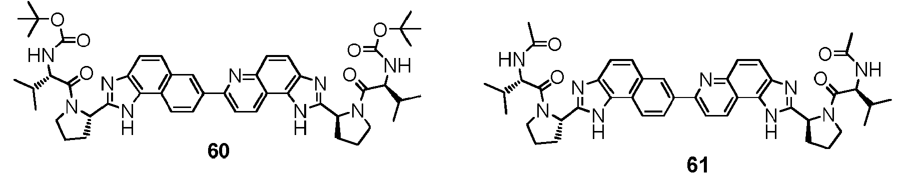 oder 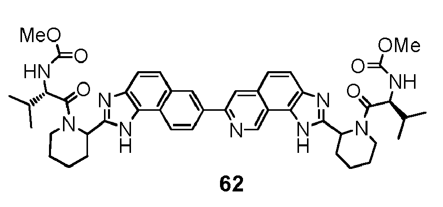 oder ein pharmazeutisch annehmbares Salz davon.

6. Eine pharmazeutische Zusammensetzung, umfassend wenigstens eine Verbindung nach Anspruch 1 oder ein pharmazeutisch annehmbares Salz oder Solvat davon und wenigstens einen pharmazeutisch annehmbaren Träger.

7. Die pharmazeutische Zusammensetzung nach Anspruch 6, ferner umfassend wenigstens ein zusätzliches therapeutisches Mittel, wobei das wenigstens eine zusätzliche therapeutische Mittel keine Verbindung nach Anspruch 1 ist, wobei das wenigstens eine zusätzliche therapeutische Mittel ausgewählt ist aus: einem Interferon, einem Immunmodulator, einem Virusreplikationsinhibitor, einem Antisense-Mittel, einem therapeutischen Vakzin, einem Inhibitor von viraler Polymerase, einem Nukleosidinhibitor, einem Inhibitor von viraler Protease, einem Inhibitor von viraler Helikase, einem Inhibitor der Virionproduktion, einem Inhibitor des Viruseintritts, einem Inhibitor der Virusassemblierung, einer Antikörpertherapie (monoklonal oder polyklonal) und einem Mittel, das sich zur Behandlung einer RNA-abhängigen polymerasebezogenen Störung eignet.

8. Eine Verbindung nach Anspruch 1 oder ein pharmazeutisch annehmbares Salz oder Solvat davon zur Verwendung bei der Behandlung einer Virusinfektion bei einem Patienten.

9. Die Verbindung zur Verwendung nach Anspruch 8, ferner umfassend die Verabreichung von wenigstens einem zusätzlichen therapeutischen Mittel an den Patienten, wobei das wenigstens eine zusätzliche therapeutische Mittel keine Verbindung nach Anspruch 1 ist, wobei das wenigstens eine zusätzliche therapeutische Mittel ausgewählt ist aus: einem Interferon, einem Immunmodulator, einem Virusreplikationsinhibitor, einem Antisense-Mittel, einem therapeutischen Vakzin, einem Inhibitor von viraler Polymerase, einem Nukleosidinhibitor, einem Inhibitor von viraler Protease, einem Inhibitor von viraler Helikase, einem Inhibitor der Virionproduktion, einem Inhibitor des Viruseintritts, einem Inhibitor der Virusassemblierung, einer Antikörpertherapie (monoklonal oder polyklonal) und einem Mittel, das sich zur Behandlung einer RNA-abhängigen polymerasebezogenen Störung eignet.

## Revendications

1. Composé répondant à la formule (I) : 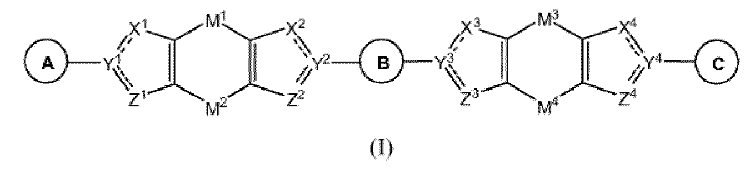 ou sel ou solvate pharmaceutiquement acceptable de celui-ci, dans lequel le groupe : 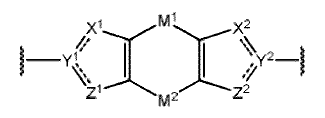 présente la structure : 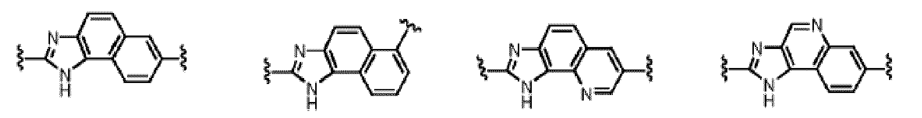 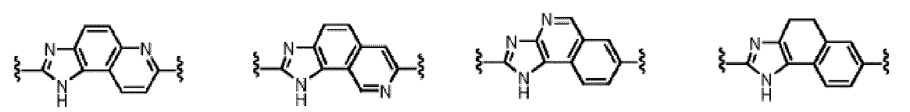 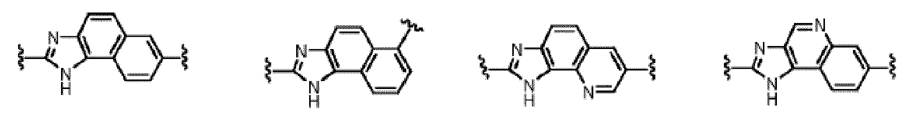 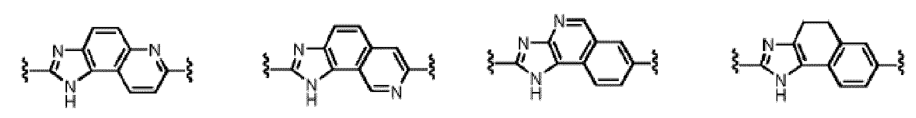 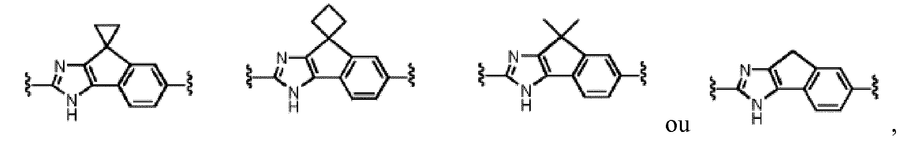 le groupe : 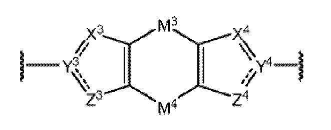 présente la structure : 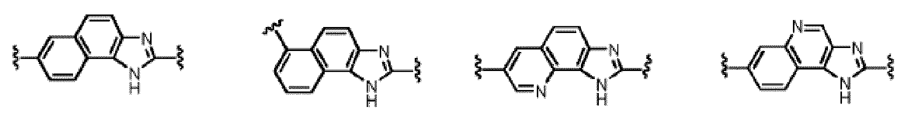 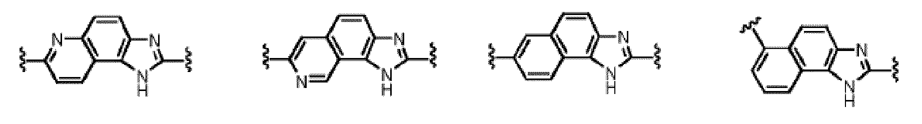 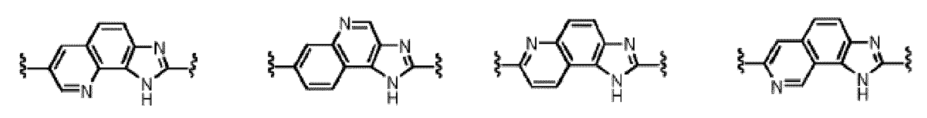 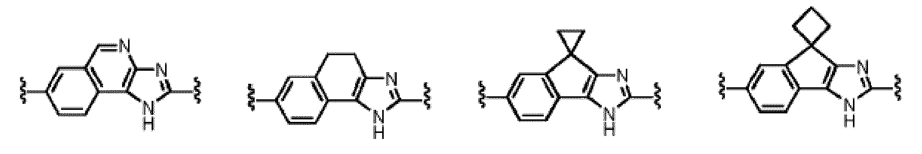 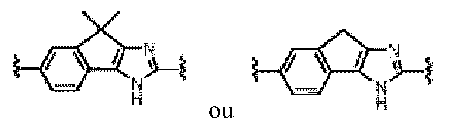 A et C représentent chacun : 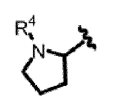 où, dans chaque cas, R⁴ est indépendamment : 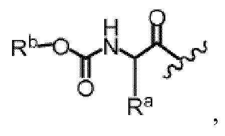 où R^{a} représente un atome de H, un groupe alkyle, halogénoalkyle, cycloalkyle ou aryle, et R^{b} représente un groupe alkyle, halogénoalkyle ou cycloalkyle, et
B représente une liaison ou bien un groupe phénylène ;
de manière qu'au moins l'un des cycles contenant : (i) X², Y² et Z² ou (ii) X³, Y³ et Z³ soit différent d'un cycle benzène.

2. Composé selon la revendication 1, dans lequel, dans chaque cas, R⁴ est indépendamment choisi parmi :

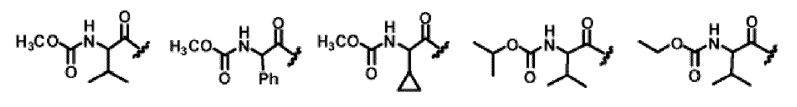

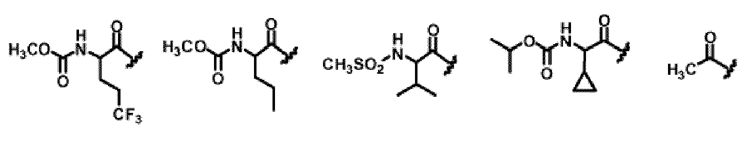

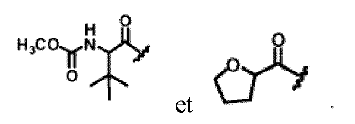

3. Composé selon la revendication 2, dans lequel dans chaque cas, R⁴ représente :

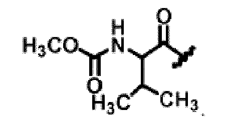

4. Composé selon la revendication 3, dans lequel B est une liaison.

5. Composé selon la revendication 1, présentant la structure : 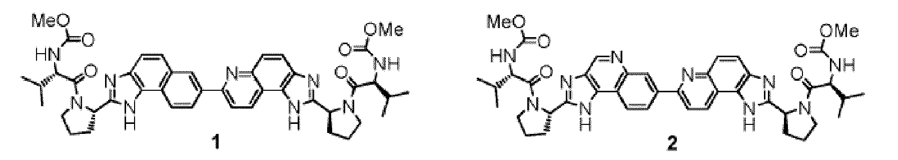 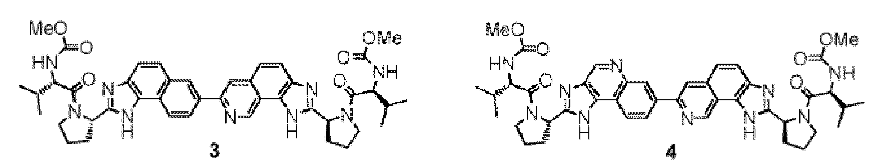 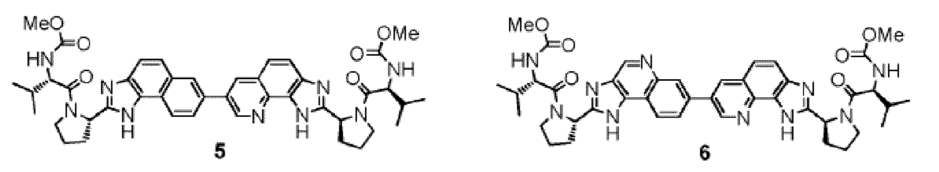 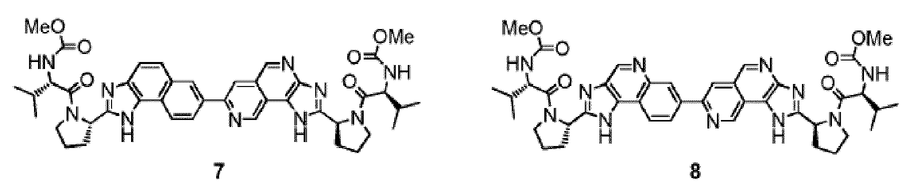 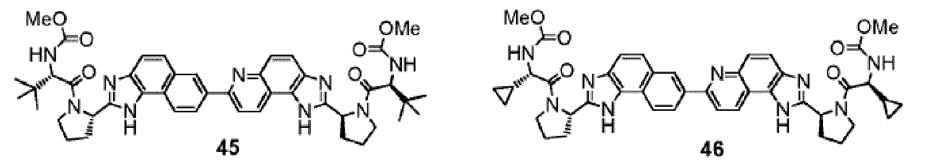 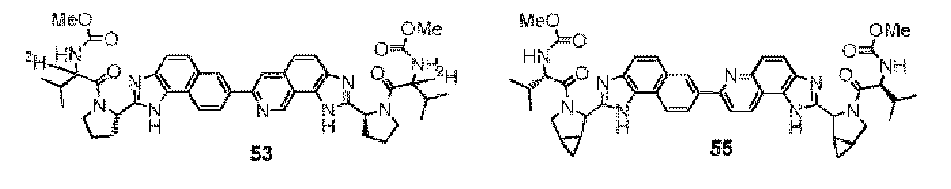 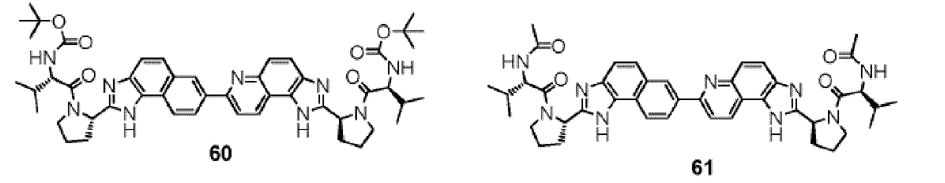 ou 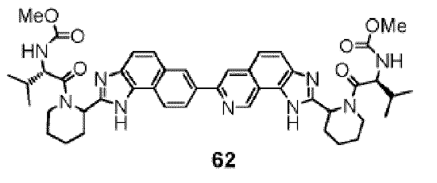 ou sel ou solvate pharmaceutiquement acceptable de celui-ci.

6. Composition pharmaceutique comprenant au moins un composé selon la revendication 1 ou un sel ou solvate pharmaceutiquement acceptable de celui-ci, et au moins un véhicule pharmaceutiquement acceptable.

7. Composition pharmaceutique selon la revendication 6, comprenant en outre au moins un agent thérapeutique additionnel, dans lequel l'au moins un agent thérapeutique additionnel n'est pas un composé selon la revendication 1, l'au moins un agent thérapeutique additionnel étant choisi parmi : un interféron, un immunomodulateur, un inhibiteur de la réplication virale, un agent anti-sens, un vaccin thérapeutique, un inhibiteur de la polymérase virale, un inhibiteur nucléosidique, un inhibiteur de la protéase virale, un inhibiteur de l'hélicase virale, un inhibiteur de la production de virions, un inhibiteur de la pénétration virale, un inhibiteur de l'assemblage viral, une thérapie par des anticorps (monoclonaux ou polyclonaux), et tout agent quelconque utile dans le traitement d'un trouble associé à une polymérase ARN-dépendante.

8. Composé selon la revendication 1, ou sel ou solvate pharmaceutiquement acceptable de celui-ci, destiné au traitement d'une infection virale chez un patient.

9. Composé destiné à une utilisation selon la revendication 8, comprenant en outre l'administration au patient d'au moins un agent thérapeutique additionnel, dans lequel l'au moins un agent thérapeutique additionnel n'est pas un composé selon la revendication 1, l'au moins un agent thérapeutique additionnel étant choisi parmi : un interféron, un immunomodulateur, un inhibiteur de la réplication virale, un agent anti-sens, un vaccin thérapeutique, un inhibiteur de la polymérase virale, un inhibiteur nucléosidique, un inhibiteur de la protéase virale, un inhibiteur de l'hélicase virale, un inhibiteur de la production de virions, un inhibiteur de la pénétration virale, un inhibiteur de l'assemblage viral, une thérapie par des anticorps (monoclonaux ou polyclonaux), et tout agent quelconque utile dans le traitement d'un trouble associé à une polymérase ARN-dépendante.
